# EUROPÄISCHE PATENTSCHRIFT

(11) **EP 3 412 234 B1**
(45) Veröffentlichungstag und Bekanntmachung des Hinweises auf die Patenterteilung: **16.11.2022**
(21) Anmeldenummer: 17174743.9
(22) Anmeldetag: 07.06.2017
(51) Int. Cl.: A61B 18/04

(54) **INSTRUMENT MIT EINEM MEHRSTROMINSTRUMENTENKOPF ZUR ARGON-PLASMA-KOAGULATION**
INSTRUMENT WITH A MULTI-FLOW INSTRUMENT HEAD FOR ARGON-PLASMA COAGULATION
INSTRUMENT COMPRENANT UNE TÊTE MULTI-INSTRUMENTS ÉLECTRIQUES POUR LA COAGULATION PLASMA D'ARGON

(43) Veröffentlichungstag der Anmeldung: 12.12.2018
(73) Patentinhaber: Erbe Elektromedizin GmbH, 72072 Tübingen (DE)
(72) Erfinder: Brodbeck, Achim, 72555 Metzingen (DE); Herrberg, Charlotte, 72411 Bodelshausen (DE); Stäbler, thomas, 72070 Tübingen (DE)
(74) Vertreter: Rüger Abel Patentanwälte PartGmbB

(56) Entgegenhaltungen:
- EP-A1- 1 090 597
- EP-A1- 3 141 203
- DE-A1-102005 021 304
- GB-A- 2 495 400
- JP-A- 2002 301 088
- US-A1- 2004 044 342
- US-A1- 2008 039 834
- US-A1- 2009 121 637
- US-A1- 2011 288 547

## Beschreibung

Die Erfindung betrifft ein Instrument für die Argon-Plasma-Koagulation.

Bei einer monopolaren Argon-Plasma-Koagulation (APC) wird das distale Ende eines Instruments in die Nähe des zu behandelnden Gewebes gebracht und zwischen einer Elektrode an dem distalen Ende des Instruments und der Gewebeoberfläche wird ionisiertes Argongas gezündet, so dass sich in der Folge ein Plasmastrahl ausbildet. Mit dem Plasmastrahl kann ein thermischer Gewebeeffekt ohne Kontakt des distalen Endes des Instruments mit dem Gewebe erzeugt werden. Insbesondere kann das Gewebe damit devitalisiert werden oder an dem Gewebe kann eine Blutstillung vorgenommen werden. Der Zündabstand bezeichnet den Abstand, bis zu dem das Instrument an das Gewebe herangeführt werden muss, so dass das Zünden des Plasmas gelingt.

Wünschenswert ist ein großer Zündabstand, um ein zuverlässiges Zünden eines Plasmas auch bei größeren Abständen zu gewährleisten. Der Zündabstand sollte auch unter zündabstandreduzierenden Einflüssen, bspw. Flüssigkeit oder Feuchtigkeit im Arbeitsbereich, groß sein. Ein großer Zündabstand ermöglicht auch bei Veränderungen des Abstands zwischen dem distalen Ende des Instruments und dem Gewebe eine kontinuierliche Behandlung. Dies ist insbesondere für eine kontinuierliche APC-Behandlung großflächiger Gewebeareale von Vorteil, da sich während der Anwendung der Abstand zum Gewebe und die lokalen Zündbedingungen stark ändern können. Ein Instrument mit großem Zündabstand muss während der Behandlung unterschiedlicher Abschnitte des Gewebes nicht so häufig neu zum Gewebe positioniert werden, da mit einem Instrument mit großem Zündabstand Bedingungen zum Zünden eines Plasmas in einem großen Bereich an Positionen des Instruments relativ zu dem Gewebe bestehen. Wenn das Instrument in einem großen Abstand über das Gewebe geführt wird, kann mit dem Plasma ein großflächiger Gewebebereich zugleich behandelt werden. Zudem verbessert ein vergrößerter Abstand des distalen Endes des Instruments zu dem zu behandelnden Gewebe während der Behandlung die Sicht auf das zu behandelnde Gewebe für den Benutzer des Instruments, beispielsweise durch eine Bildübertragungseinrichtung am distalen Ende eines Endoskops, an dem oder in dem das Instrument befestigt sein kann.

Bei bekannten Instrumenten nimmt deren Zündabstand zum Gewebe relativ zum Einsatz des Instruments in trockener Umgebung beim Einsatz in feuchter bzw. nasser Umgebung spürbar ab, was dazu führt, dass das Instrument zum Zünden näher an das zu behandelnde Gewebe herangeführt werden muss. Dadurch kann die mit dem Instrument pro Zeiteinheit behandelbare Gewebefläche (Flächenleistung) auf Grund der öfter erforderlichen Neuposition des Instruments zum Gewebe abnehmen. Der Zeitbedarf für eine Behandlung ist dann entsprechend erhöht. Außerdem kann die freie Sicht auf das Behandlungsgebiet infolge von Abschattung durch das Instrument eingeschränkt sein. Zudem besteht bei nahem Abstand eine erhöhte Wahrscheinlichkeit die Spitze des Instruments mit Gewebe zu verschmutzen.

Aus der DE 10 2005 021 304 A1 ist eine endoskopische Chirurgieeinrichtung für eine Argon-Plasma-Koagulation bekannt, die eine in ein Endoskop einführbare Arbeitseinrichtung aufweist, die über einen ersten Kanal verfügt, in dem eine Elektrode angeordnet ist und durch den Inertgas an das Gewebe herangeführt werden kann. Die Arbeitseinrichtung weist auch einen zweiten Kanal auf. Dieser dient dazu, vor der Aktivierung des HF-Stromes und während der eigentlichen APC Argongas an das Operationsgebiet heranzuführen, um brennbare Gase wie z.B. Sauerstoff oder Kohlenmonoxid von dem Operationsgebiet zu verdrängen bzw. fernzuhalten.

In dem Beitrag "Challenges and solutions on the way to a deployable plasma endoscope" auf der 6th International Conference on Plasma Medicine (ICPM 6), September 4-9, 2016, Bratislava, Slowakei von J. Winter et al. wurde eine Vorrichtung zur endoskopischen Plasmatherapie vorgestellt. Es wird ein Instrument beschrieben, dass einen Kernstrom aus Helium und einen Mantelstrom aus Luft erzeugt. Damit sollen Nachteile bei der Plasmaerzeugung durch Anreicherung von Inertgas in einem Hohlkörper vermieden werden.

EP 3 141 203 A1 offenbart eine Ablationsvorrichtung mit einem Kopf, in welchem zwei Kanäle ausgebildet sind, die in einem Winkel zueinander angeordnet sind und Elektroden enthalten. Dadurch kann ein fächerförmiger Plasmastrahl erzeugt werden, um einen breiten streifenförmigen Ablationsbereich zu erzielen.

JP 2002-301088 A zeigt eine Sonde zur Koagulation, deren Sondenkopf mehrere Öffnungen aufweist und ein Fluid und Hochfrequenzstrom gleichzeitig in unterschiedliche Richtungen abgeben soll.

GB 2495400 A zeigt ein elektrochirurgisches Instrument mit einem Kopf mit mehreren Öffnungen an der Spitze. Der Kopf weist außerdem seitliche Öffnungen auf. In dem Kopf endet eine Elektrode. Je nach dem welche der Öffnungen am nächsten am Gewebe angeordnet ist, soll das Gas, das diese Öffnung verlässt, durch die Elektrode in einen Plasmazustand überführt werden.

US 2011/0288547 A1 offenbart eine Sonde, in deren Kopf zwei Elektroden angeordnet sind, wobei der Kopf seitliche Öffnungen aufweist.

Die Sonde der US 2008/0039834 A1 weist ein distales Ende mit einer distalen Öffnung und einem Gasauslass auf, durch welchen Gas aus dem distalen Ende ausströmen kann, wenn der Benutzer des Instruments das distale Ende mit der Öffnung auf Gewebe aufsetzt.

US 2004/0044342 A1 offenbart eine Sonde mit Auslässen, welche dem Gasablass dienen, wenn die Öffnung am distalen Ende der Sonde auf Gewebe gedrückt wird.

Die Vorrichtung zur Erzeugung von Plasma gemäß US 2009/0121637 A1 offenbart eine dielektrische Röhre und in der Röhre angeordnete dielektrische Scheiben mit Öffnungen und Ringelektroden, welche die Öffnungen umgeben.

Die Koagulationssonde der EP 1 090 597 A1 weist mehrere Öffnungen auf, denen Elektroden zugeordnet sind, die mit elektrischer Leistung gespeist werden können, um Plasmaströme aus den Öffnungen zu erzeugen.

Aufgabe der vorliegenden Erfindung ist, ein Instrument zur elektrochirurgischen Behandlung von Gewebe mit einem großen Zündabstand anzugeben.

Diese Aufgabe wird mit einem elektrochirurgischen Instrument nach Anspruch 1 gelöst:
Das erfindungsgemäße elektrochirurgische Instrument weist einen Kopf mit einem mit Gas, insbesondere Inertgas, bspw. Argon, beaufschlagbaren Lumen auf, das zu einem Kanal gehört. Der Kopf weist ferner wenigstens eine an das Lumen angeschlossene erste Öffnung, die zur Ausbildung eines Primärstroms an Gas (Primärgasstrom) vor oder neben dem distalen Ende des Instruments, außerhalb des Endes des Instruments, von dem distalen Ende des Instruments weg dient, und eine oder mehrere an dasselbe Lumen angeschlossene zweite Öffnungen zur Ausbildung eines Sekundärstroms an Gas (Sekundärgasstrom) neben dem Primärstrom oder, zumindest teilumfänglich, um den Primärstrom herum auf. In dem Kopf ist eine Elektrode angeordnet. Die Elektrode ist in dem Kopf vorzugsweise derart angeordnet, dass das Gas für den Primärstrom bei Beaufschlagung des Lumens mit Gas an der Elektrode vorbeiströmt. Die Elektrode ist in einem an die erste Öffnung angrenzenden, in die erste Öffnung mündenden Kanalabschnitt des Kanals angeordnet.

Wenn das Lumen mit Gas, insbesondere Inertgas, beispielsweise Argon, beaufschlagt wird, strömt das Gas durch das Lumen einerseits zu der ersten Öffnung und andererseits zu der zweiten Öffnung und aus diesen Öffnungen heraus. Aus dem Lumen durch die Öffnungen strömt somit das gleiche Gas. Mit Hilfe der Elektrode kann ein Plasma zur Behandlung des Gewebes vor der ersten Öffnung gezündet werden.

Das Instrument ist dazu ausgebildet, dass der Sekundärstrom, vorzugsweise zumindest abschnittsweise umfänglich um den Primärstrom, zwischen dem Primärstrom und einem Bereich der Umgebung des Instrumentenkopfes ausgebildet wird. Der Bereich kann beispielsweise feuchtes Gas, z.B. feuchte Luft oder feuchtes Inertgas oder Mischungen davon, Aerosol und/oder Flüssigkeit enthalten. Es erfolgt entsprechend eine Trennung des Primärstroms quer zur Strömungsrichtung des Primärstroms von dem Bereich der Umgebung durch den Sekundärstrom, der zwischen dem Primärstrom und dem Bereich der Umgebung strömt. Die Trennung erfolgt vorzugsweise zumindest abschnittsweise umfänglich um den Primärstrom.

Es wird angenommen, dass die Ausbildung eines Sekundärstroms neben oder, zumindest teilumfänglich, um den Primärstrom herum einerseits zu einer Stabilisierung des Primärstroms durch den Sekundärstrom führt und andererseits dazu führt, dass Verschmutzungen, Feuchtigkeit oder Flüssigkeitstropfen von der ersten Öffnung von dem Sekundärstrom weggetragen oder weggeblasen werden. In jedem Fall weist das erfindungsgemäße Instrument einen großen Zündabstand auf.

Das erfindungsgemäße Instrument ist sowohl für offenchirurgische Eingriffe als auch für endoskopische Eingriffe geeignet. Insbesondere bei letzteren besteht eine erhöhte Gefahr des Anhaftens von Feuchtigkeit oder Flüssigkeit an dem distalen Ende des Instruments und eine in der Regel feuchte Umgebung um den Instrumentenkopf während des Einsatzes des Instruments.

Dadurch, dass die erste Öffnung und die zweite Öffnung von demselben Lumen in dem Kopf des Instruments gespeist werden, müssen nicht zwei gesonderte Kanäle an das Instrumentenende herangeführt werden. Vielmehr kann eine Gasleitung mit einem einzigen, zwischen dem proximalen Ende der Gaszuführungsleitung an der Gasversorgungseinrichtung und dem Lumen unverzweigten Kanal zum Zuführen von Gas in das Lumen verwendet werden. Es ergibt sich die Möglichkeit zur Gestaltung eines schlanken Instruments mit einem schlanken Kopf, das sich insbesondere zum Einsatz mit einem Endoskop eignet, wenn das Instrument außen an einem Endoskop befestigt oder in einem Arbeitskanal eines Endoskops geführt wird.

Das erfindungsgemäße Instrument kann durch ein oder mehrere der nachfolgend beschriebenen Merkmale oder ein oder mehrere der in den Zeichnungen gezeigten Merkmale weitergebildet werden.

Als vorteilhaft hat sich erwiesen, wenn die zweite Öffnung von der ersten Öffnung zurückversetzt angeordnet ist. Wenn die zweite Öffnung gegenüber der ersten Öffnung zurückversetzt angeordnet ist, wird zwischen der von der ersten Öffnung zurückversetzt angeordneten zweiten Öffnung und der ersten Öffnung ein Abstand zur Ausbildung eines außerhalb des Instrumentenkopfes strömenden Sekundärstroms geschaffen, der seitlich neben der ersten Öffnung an der ersten Öffnung vorbeiströmt, bereitgestellt und Feuchtigkeit und Tropfen können damit besonders effektiv von dem Sekundärstrom von dem Bereich des Instrumentenkopfes um die ersten Öffnung herum entfernt werden.

Der Kanal kann einen an die Öffnung angrenzend angeschlossenen Primärstromkanalabschnitt aufweisen, der einen Unterabschnitt des Kanalabschnitts bildet, in dem die Elektrode angeordnet ist. Der Primärstromabschnitt des Kanals ist zwischen der ersten Öffnung und dem Lumen angeordnet, um die erste Öffnung mit dem Lumen zu verbinden. Bei Beaufschlagung des Instrumentenkopfes mit Gas tritt das Gas aus dem Lumen durch den Primärstromkanalabschnitt hindurchund anschließend aus der erste Öffnung heraus. In einer Ausführungsform kann sich der Strömungsquerschnitt des Primärstromkanalabschnitts vor der ersten Öffnung in Richtung zu der ersten Öffnung verjüngen. Der von der vor der ersten Öffnung in Richtung zu der ersten Öffnung abnehmende Strömungsquerschnitt führt zu einer Beschleunigung des Primärstroms.

In dem Instrumentenkopf ist neben dem Primärstromkanalabschnitt oder zumindest abschnittsweise umfänglich, um den Primärstromkanalabschnitt vorzugsweise wenigstens ein Sekundärstromkanalabschnitt des Kanals ausgebildet, der an die zweite Öffnung angrenzend angeschlossen ist, um die zweite Öffnung mit dem Lumen zu verbinden. Mittels des Sekundärstromkanalabschnitts kann der durch den Sekundärstromkanalabschnitt strömende Gasstrom geführt, ausgerichtet und/oder beschleunigt werden.

Der an das Lumen angeschlossene Eingang des Primärstromkanalabschnitts kann gegenüber der zweiten Öffnung nach proximal und/oder in Richtung entgegen die Strömungsrichtung durch den Primärstromkanalabschnitt zurückversetzt sein. Dadurch können ein oder mehrere Sekundärstromkanalabschnitte neben dem Primärstromkanalabschnitt oder zumindest teilumfänglich um den Primärstromkanalabschnitt gebildet sein. Zudem ist durch das zurückversetze Anordnen des Eingangs der Ort des Aufteilens des durch das Lumen hindurchtretenden Gasstroms von dem Ort des Austritts aus der zweiten Öffnung und dem Ort des Austritts aus der ersten Öffnung getrennt.

Der Eingang des Primärstromkanalabschnitts kann eine Aufteilungskante aufweisen, die den Gasstrom in einen Primärstrom durch den Primärstromkanalabschnitt und einen Sekundärstrom durch die zweite Öffnung aufteilt. Die Aufteilungskante ist kann beispielsweise schneidenförmig sein.

Die erste Öffnung kann beispielsweise rundsymmetrisch, insbesondere kreisförmig, sein. Die Öffnungsbreite der ersten Öffnung ist vorzugsweise größer als die orthogonal zur Öffnungsbreite gemessene Öffnungshöhe der ersten Öffnung. Die zweiten Öffnungen sind vorzugsweise auf sich entlang der Öffnungsbreite der ersten Öffnung erstreckenden Seiten des Instrumentenkopfes angeordnet. Die erste Öffnung kann beispielsweise abgeflacht gerundet sein. Die erste Öffnung kann beispielsweise oval sein. Die erste Öffnung kann bis auf einen Schlitz abgeflacht sein. Die erste Öffnung weist vorzugsweise eine Form auf, um einen fächerförmigen Primärstrom zu erzeugen.

Die zweite Öffnung ist vorzugsweise nierenförmig, bogenförmig, sichelförmig oder schlitzförmig. Die zweite Öffnung ist vorzugsweise derart angeordnet, um einen Sekundärstrom zu erzeugen, der den Primärstrom zumindest teilumfänglich, im Querschnitt beispielsweise nierenförmig, bogenförmig oder sichelförmig umgibt. Um einen den Primärstrom wenigstens teilumfänglich umgebenden Sekundärstrom zu erzeugen, kann die zweite Öffnung bspw. nierenförmig, bogenförmig oder sichelförmig um die erste Öffnung bzw. den Primärstromkanalabschnitt angeordnet sein.

In Strömungsrichtung hinter der zweiten Öffnung an dem Kopf des Instruments kann ein über die zweite Öffnung hinaus vorstehendes Teil angeordnet sein, so dass der die zweite Öffnung verlassende Sekundärstrom zwischen dem vorstehenden Teil und dem Primärstrom strömt. Das vorstehende Teil kann beispielsweise ein Abschnitt des Kopfes sein oder es kann ein an dem Instrument befestigtes, von dem Instrument gesondertes Teil sein. Das vorstehende Teil zögert eine seitliche Expansion des aus der zweiten Öffnung herausgetretenen Sekundärstroms hinaus und kann somit zu einem großen Zündabstand des Instruments beitragen.

Das Instrument ist vorzugsweise zur Ausbildung eines Sekundärstroms neben dem oder um den Primärstrom herum derart eingerichtet, dass der Sekundärstrom gemessen an der ersten Öffnung eine höhere Strömungsgeschwindigkeit aufweist als der Primärstrom gemessen an der ersten Öffnung. Dies kann beispielsweise durch Beschleunigung des Sekundärstroms und/oder durch Anordnung von die Strömungsgeschwindigkeit des Gases für den Primärstrom vermindernden Elementen im Inneren des Kopfteils erreicht werden.

Der Strömungsquerschnitt des Sekundärstromkanalabschnitts vor der zweiten Öffnung kann sich in Richtung zu der zweiten Öffnung verjüngen. Durch die in Strömungsrichtung zu der zweiten Öffnung abnehmende Strömungsquerschnittsfläche des Sekundärstromkanalabschnitts kann das durch den Sekundärstromkanalabschnitt in Richtung zu der zweiten Öffnung strömende Gas des Sekundärstroms vor dem Austritt aus der zweiten Öffnung beschleunigt werden. Das Instrument kann zur Beschleunigung des Sekundärstroms beispielsweise derart eingerichtet sein, dass der Sekundärstrom gemessen an der ersten Öffnung eine höhere Strömungsgeschwindigkeit aufweist als der Primärstrom gemessen an der ersten Öffnung.

Das Instrument ist, wie erwähnt, dazu eingerichtet, dass bei Beaufschlagung des Lumens mit Gas an der ersten Öffnung anhaftende Flüssigkeitstropfen von der ersten Öffnung von dem Sekundärstrom und/oder dem Primärstrom weggeblasen und/oder von dem Sekundärstrom und/oder dem Primärstrom aufgenommen und weggetragen werden. Dazu kann eine Beschleunigung des Gases des Primärstroms und/oder des Sekundärstroms vor dem Austritt durch die erste Öffnung beziehungsweise durch die zweite Öffnung beitragen. Alternativ oder zusätzlich kann die Oberfläche des Instrumentenkopfes an der ersten Öffnung vorzugsweise hydrophob ausgebildet, beispielsweise durch eine hydrophobe Beschichtung, so dass das Wegblasen von Flüssigkeit von der ersten Öffnung durch den Sekundärstrom und/oder den Primärstrom auch bei geringen Volumenströmen von Gas durch den Instrumentenkopf verbessert ist.

Vorzugsweise ist der Instrumentenkopf dazu ausgebildet, den Sekundärstrom auf den Primärstrom zu richten. Dazu können Leitflächen des Sekundärstromkanalabschnitts und/oder des vorstehenden Teils entsprechend in einem spitzen Winkel zwischen größer 0° und kleiner 90° zu der Strömungsrichtung des Primärstroms ausgerichtet ausgebildet sein.

Die Elektrode kann in der ersten Öffnung angeordnet sein, so dass das Ende der Elektrode, an dem das Plasma gebildet wird, mit der ersten Öffnung abschließt. Andernfalls kann die Elektrode beispielsweise derart an der ersten Öffnung angeordnet sein, dass das Ende in den Instrumentenkopf nach proximal zurückversetzt angeordnet ist. Die Elektrode kann vor der ersten Öffnung angeordnet sein, so dass das Gas bei Beaufschlagung des Lumens mit Gas aus dem Lumen zunächst an dem Ende der Elektrode vorbeiströmt bevor es aus der ersten Öffnung austritt. Die Elektrode kann beispielsweise so im Strömungsweg des Gases, welcher aus dem Lumen, bis zu der ersten Öffnung und aus der ersten Öffnung heraus führt, angeordnet sein, dass die Elektrode auf gegenüberliegenden Seiten der Elektrode von Gas umströmt wird.

Das elektrochirurgische Instrument weist vorzugsweise eine plättchen-, spatel-, nadel- und/oder messerförmige Elektrode auf. Solche Bauformen behindern den Gasstrom möglichst wenig. Die Elektrode kann in dem Instrumentenkopf, beispielsweise in dem Primärstromkanalabschnitt befestigt sein, indem die Elektrode in ihrer Breite ein Übermaß gegenüber dem Kanal des Instrumentenkopfes, beispielsweise gegenüber dem Primärstromkanalabschnitt, aufweist, so dass sich die Elektrode, nach der Anordnung der Elektrode in dem Kanal, auf Grund einer elastische Verformung der Elektrode auf gegenüberliegenden Seiten gegen die innere Wandfläche des Kanals stemmt.

Das Ende der Elektrode, an dem bei Beaufschlagung der Elektrode mit HF-Leistung das Plasma gebildet wird, ist erfindungsgemäß in Strömungsrichtung nach der zweiten Öffnung angeordnet. Auf diese Weise kann die Wahrscheinlichkeit eines Durchschlags eines Funkens von dem Ende der Elektrode durch die zweite Öffnung verringert werden. Vorzugsweise ist zusätzlich das gegenüberliegende, dem Plasma abgewandte Ende der Elektrode in Strömungsrichtung gesehen nach der zweiten Öffnung angeordnet, um die Wahrscheinlichkeit eines Durchschlags eines Funkens von dem proximalen Ende der Elektrode durch die zweite Öffnung zu vermindern.

Das erfindungsgemäße elektrochirurgische Instrument ist vorzugsweise ein monopolares Instrument. Bei einem solchen wird der Stromkreis von dem HF-Generator zu der Elektrode über das Plasma in das Gewebe des Patienten von einer an dem Patienten befestigten Neutralelektrode zurück in den HF-Generator geschlossen. Im Gegensatz dazu ist an einem bipolaren Instrument eine Rückführungselektrode vorhanden, wobei der Stromkreis von der Elektrode in das Plasma und aus dem Plasma zur Rückführungselektrode geschlossen wird.

Es wird zudem ein Kopf für ein elektrochirurgisches Instrument wie hierin beschrieben angegeben. Der Instrumentenkopf kann ein von einer Gaszuführungsleitung gesondertes Teil sein, das mit der Gaszuführungsleitung zur Herstellung des Instruments verbunden werden kann.

Weitere bevorzugte Merkmale des erfindungsgemäßen Instruments werden in den im Folgenden beschriebene Ausführungsformen beschrieben und in der Zeichnung veranschaulicht, deren Merkmale untereinander kombiniert werden können.

Es zeigen schematisch und beispielhaft:
Fig. 1 - ein Ausführungsbeispiel eines erfindungsgemäßes Systems mit einem beispielhaften erfindungsgemäßen Instrument in einem Lumen eines menschlichen Körpers in einer teilgeschnittenen Ausschnittsansicht,
Fig. 2a - einen Ausschnitt eines Ausführungsbeispiels eines erfindungsgemäßen Instruments in einer längsgeschnittenen Ansicht,
Fig. 2b - eine Frontalansicht auf das Instrument gemäß Fig. 2a,
Fig. 2c - eine längsgeschnittene Ausschnittsansicht des Instruments gemäß Fig. 2a mit einem Führungsteil,
Fig. 3a - einen Ausschnitt eines erfindungsgemäßen Instruments gemäß einem weiteren Ausführungsbeispiel,
Fig. 3b - das erfindungsgemäße Instrument gemäß Fig. 3a in einer Frontalansicht,
Fig. 4 - ein weiteres Ausführungsbeispiel des erfindungsgemäßen Instruments in einer längsgeschnittenen Ausschnittsansicht,
Fig. 5 - einen Ausschnitt eines weiteres Ausführungsbeispiels des erfindungsgemäßen Instruments in einer längsgeschnittenen Ansicht,
Fig. 6 - einen Ausschnitt eines Ausführungsbeispiels entsprechend Fig. 5,
Fig. 7a - ein erfindungsgemäßer Instrumentenkopf in einer seitlichen Ansicht,
Fig. 7b - ein erfindungsgemäßes Instrument mit einem Instrumentenkopf gemäß Fig. 7a,
Fig. 8a, 8b, 8c - beispielhafte Elektrodenformen in Ausführungsformen des erfindungsgemäßen Instruments,
Fig. 9a, 9b - eine beispielhafte Ausführungsform des erfindungsgemäßen Instruments,
Fig. 10 - eine beispielhafte Ausführungsform des erfindungsgemäßen Instrumentenkopfs,
Fig. 11, 12 - beispielhafte Anordnungen der zweiten Öffnungen in Ausführungsbeispielen erfindungsgemäßer Instrumente.

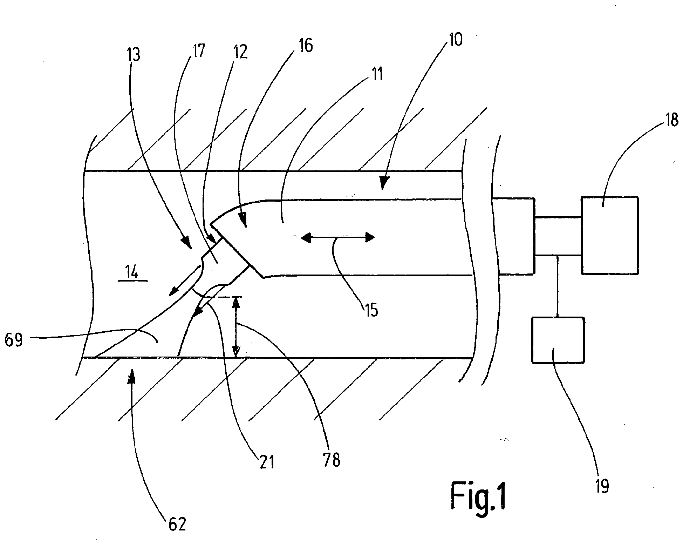

Fig. 1 zeigt eine erfindungsgemäße Vorrichtung 10 mit einem Endoskop 11 und einer sich durch einen Arbeitskanal des Endoskops 11 erstreckenden Gaszuführungsleitung 12 eines erfindungsgemäßen elektrochirurgischen Instruments 13 in einem Hohlraum 14 des Körpers eines Patienten in einer Seitenansicht auf das Instrument 13. Das erfindungsgemäße Instrument 13 kann alternativ beispielsweise außen an dem Endoskop 11 geführt sein. Das Endoskop 11 ist in Fig. 1 beispielhaft mit einem gegenüber der Längserstreckungsrichtung 15 des Endoskops 11 gekrümmten distalen Ende 16 dargestellt. Der aus dem Arbeitskanal des Endoskops 11 hervorschauende Kopf 17 des Instruments 13 (Instrumentenkopf) bildet einen distalen Endabschnitt des Instruments 13. Das Instrument 13 ist an einer in Fig. 1 ebenfalls dargestellten Gasversorgungseinheit 18 und einem HF-Generator 19 angeschlossen.

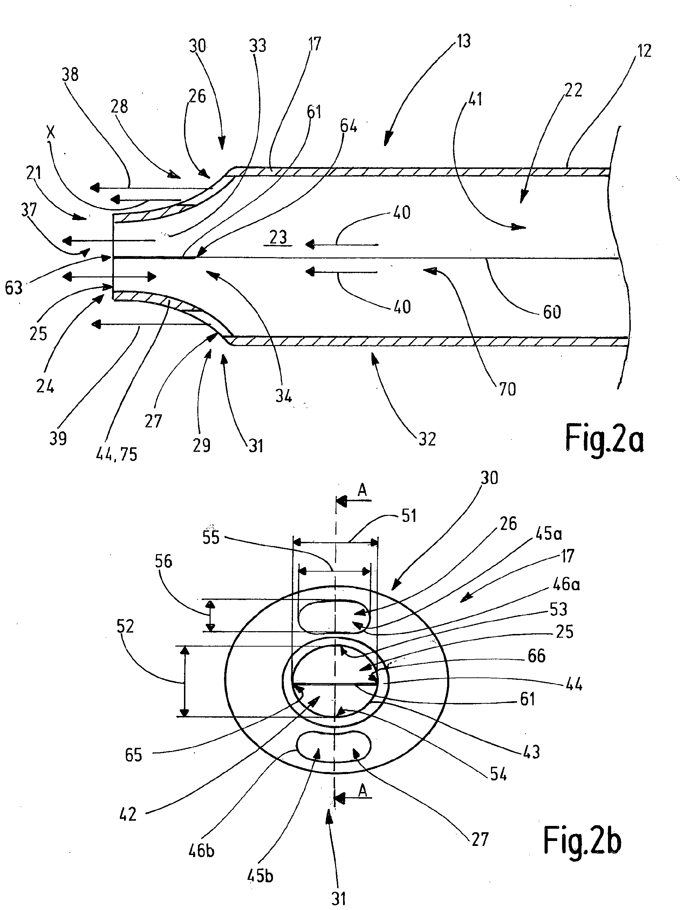

Fig. 2a zeigt einen Ausschnitt des elektrochirurgischen Instruments 13 mit dessen Kopf 17 in einer Längsschnittansicht (Längsschnittdarstellung entlang der Schnittlinie A-A in Fig. 2b). Fig. 2b zeigt den Instrumentenkopf 17 der Fig. 2a in einer Ansicht von vorne, von außerhalb des Instrumentenkopfes 17 auf die Stirnseite 21 des Instrumentenkopfes 17. Das Instrument 13 weist einen Kanal 22 auf, der an die Gasversorgungseinheit 18 angeschlossen ist. Zu dem Kanal 22 gehört ein Lumen 23 (Hohlraum) in dem Instrumentenkopf 17, das fluidisch an eine in einem zentralen Bereich 24 der Stirnseite 21 angeordnete erste Öffnung 25 und zwei zweite Öffnungen 26, 27 angeschlossen ist, die in peripheren Bereichen 28, 29 der Stirnseite 21 um den zentralen Bereich 24 angeordnet sind. Im dargestellten Ausführungsbeispiel sind die zweiten Öffnungen 26, 27 auf gegenüberliegenden Seiten 30, 31 des Instrumentenkopfes 17 ausgebildet. Während die erste Öffnung 25 und die zweite Öffnung 26 in dem in Fig. 2a und 2b dargestellten Ausführungsbeispiel in einer Stirnseite 21 des Instrumentenkopfes angeordnet sind, können die erste Öffnung 25 und die zweiten Öffnungen 26, 27 alternativ an einer Mantelfläche 32 des Instrumentenkopfes 17 angeordnet sein.

Zwischen dem Lumen 23 und der ersten Öffnung 25 ist ein zentraler Primärstromkanalabschnitt 33 des Instrumentenkopfes 17 angeordnet, der das Lumen 23 mit der ersten Öffnung 25 fluidisch verbindet.

Die zweiten Öffnungen 26, 27 sind jeweils seitlich an dem Eingang 34 in den Primärstromkanalabschnitt 33 an gegenüberliegenden Seiten des Eingangs 34 angeordnet.

Die zweite Öffnung 26, 27 und die ersten Öffnung 25 sind derart angeordnet, so dass auf zwei gegenüberliegenden Seiten neben dem Primärstrom 37 aus der ersten Öffnung 25 heraus strömende Sekundärströme 38, 39 ausgebildet werden.

Wenn die zweiten Öffnungen 26, 27, wie dargestellt, von der ersten Öffnung 25 entgegen der Strömungsrichtung 40 durch das Lumen 23, nach proximal zurückversetzt angeordnet sind, wird, in Strömungsrichtung 40 gesehen, ein Abstand x zwischen der ersten Öffnung 25 einerseits und den zweiten Öffnungen 26, 27 andererseits ausgebildet. Durch die nach proximal zurückversetzten zweiten Öffnungen 26, 27 werden neben der ersten Öffnung 25 Sekundärströme 38, 39 von Gas ausgebildet, die einen Strömungsweg entsprechend des Abstands x von den zweiten Öffnungen 26, 27 weg zurückgelegt haben. Die zweiten Öffnungen 26, 27 sind vorzugsweise jeweils um den gleichen Abstand x gegenüber der ersten Öffnung 25 entgegen der Strömungsrichtung 40 zu der ersten Öffnung zurückversetzt angeordnet. Der Abstand x kann beispielsweise bis maximal 5 Millimeter, bevorzugt bis maximal 2 Millimeter betragen. In einem Ausführungsbeispiel kann der Abstand x beispielsweise 1,2 Millimeter betragen.

Die Strömungsrichtung 40 ist die Richtung in der Gas durch das Lumen 23 strömt, wenn der Instrumentenkopf 17 an seinem proximalen Ende 41 mit Gas beaufschlagt wird, das dadurch in Strömungsrichtung 40 durch das Lumen 23 und zu der ersten Öffnung 25 und den zweiten Öffnungen 26, 27 strömt.

Die Öffnungsfläche 42, durch die das Gas aus der ersten Öffnung 25 tritt, wird von einer Endkante 43 der Wand 44 des Kanals 22 in dem Instrumentenkopf 17, hier einer Endkante 43 der Wand 44 des Primärstromkanalabschnitts 33, umgrenzt. Die Öffnungsfläche 42 der ersten Öffnung 25 steht vorzugsweise senkrecht zu der Strömungsrichtung 40 des Primärstroms durch den Primärstromkanalabschnitt 33. Die Öffnungsflächen 45a, 45b der zweiten Öffnungen 26, 27 werden jeweils von einer äußeren Kante 46a, 46b der die Öffnung 26, 27 bildenden Ausnehmung aus der Wand 44 des Kanals 22 durch den Instrumentenkopf 17 umgrenzt. Wie dargestellt sind die Öffnungsflächen 45a, 45b der zweiten Öffnungen 26, 27 jeweils schräg zur Strömungsrichtung 40 zu der ersten Öffnung 25 orientiert. Die Öffnungsflächen 45a, 45b der an dem Umfang des Instrumentenkopfes 17 angeordneten zweiten Öffnungen 26, 27 weisen jeweils in den Raum außerhalb des Instrumentenkopfes 17 an der Stirnseite 21 des Instruments 13.

Wie aus Fig. 2b erkennbar, hat die erste Öffnung 25 in dem Ausführungsbeispiel eine abgeflacht gerundete Form, um die Ausbildung eines Primärstroms 37 zu fördern, dessen Erstreckung senkrecht zur Strömungsrichtung des Primärstroms 37 gemessen in einer ersten Dimension größer ist als dessen Erstreckung senkrecht zur Strömungsrichtung des Primärstroms 37 gemessen in einer zu der ersten Dimension senkrechten zweiten Dimension. Dazu ist die Öffnungsbreite 51 der ersten Öffnung 25 größer als die senkrecht dazu gemessene Öffnungshöhe 52. Die erste Öffnung 25 weist vorzugsweise eine ovale Form auf. Alternativ kann die erste Öffnung 25 bspw. rechteckig, kreisrund oder quadratisch sein. Die erste Öffnung 25 kann bis zu einem Schlitz zur Ausbildung einer Schlitzdüse zur Erzeugung eines fächerartigen Gasstroms abgeflacht gestaltet sein. Beispielsweise kann die Öffnungsbreite 51 der ersten Öffnung 25 einen Betrag von größer 0 bis maximal zu dem 2,5-fachen des Durchmessers eines Schlauches aufweisen, der die Gaszuführungsleitung 12 bilden kann. Beispielsweise kann die Öffnungshöhe 52 der ersten Öffnung 25 einen Betrag von größer 0 bis maximal zu dem 2,5-fachen des Durchmessers des Schlauches aufweisen. Die Öffnungsbreite 51 der ersten Öffnung 25 kann beispielsweise zwischen minimal 3 bis maximal 4 Millimeter betragen. Die Öffnungshöhe 52 der ersten Öffnung 25 kann beispielsweise zwischen minimal 0,7 bis maximal 2 Millimeter betragen.

Wenn eine Elektrode mit einem flächigen Abschnitt in dem Kopf 17 angeordnet ist, ist die Anordnung der zweiten Öffnungen 26, 27 ist vorzugsweise symmetrisch bzgl. der Fläche des Abschnitts. Die zweiten Öffnungen 26, 27 sind vorzugsweise symmetrisch um den Eingang 34 des Primärstromkanalabschnitts 33 angeordnet. Die zweiten Öffnungen 26, 27 sind wie dargestellt vorzugsweise derart angeordnet, dass sich Sekundärströme 38, 39 ausbilden, die an gegenüberliegenden Seiten 53, 54 der ersten Öffnung 25 vorbeiströmen, welche Seiten 53, 54 sich entlang der Öffnungsbreite 51 der ersten Öffnung 25 erstrecken (lange Seiten 53, 54). Wenn die, beispielsweise nierenförmigen, bogenförmigen, sichelförmigen oder gekrümmt schlitzförmigen, zweiten Öffnungen 26, 27 so um den Primärstromkanalabschnitt 33, z.B. dessen Eingang 34, oder die erste Öffnung 25 angeordnet sind, dass die zweiten Öffnungen 26, 27 Sekundärströme 38, 39 erzeugen, die den Primärstrom 37 teilumfänglich umschließen und die an Wandbereichen an gegenüberliegenden langen Seiten 53, 54 der ersten Öffnung 25 vorbeiströmen, können Feuchtigkeit und/oder Flüssigkeit von den Wandbereichen an den langen Seiten 53, 54 der ersten Öffnung 25 weggeblasen oder weggetragen werden.

Die Öffnungsbreite 55 der zweiten Öffnungen 26, 27 kann, wie dargestellt, kleiner sein als die Öffnungsbreite 51 der ersten Öffnung 25. Beispielsweise kann die Öffnungsbreite 55 der zweiten Öffnungen 26, 27 jeweils einen Betrag von größer 0 bis maximal zu dem 2,5-fachen des Durchmessers eines Schlauches aufweisen, der die Gaszuführungsleitung 12 bilden kann. Beispielsweise kann die Öffnungshöhe 56 der zweiten Öffnung 26, 27 jeweils einen Betrag von größer 0 bis maximal zu dem 1,25-fachen des Durchmessers des Schlauches aufweisen. Die Öffnungsbreite 55 der zweiten Öffnungen 26, 27 kann beispielsweise zwischen minimal 1,5 bis maximal 3,5 Millimeter betragen. Die senkrecht dazu gemessene Öffnungshöhe 56 der zweiten Öffnungen 26, 27 kann beispielsweise zwischen minimal 0,1 bis maximal 0,5 Millimeter betragen.

Die Beträge der Öffnungsbreiten 55 der zweiten Öffnungen 26, 27 und/oder die Beträge der Öffnungshöhen 56 der zweiten Öffnungen 26, 27 sind vorzugsweise gleich. Die Öffnungsflächeninhalte der zweiten Öffnungen 26, 27 sind vorzugsweise gleich. Die Öffnungsfläche der ersten Öffnung 25 ist im dargestellten Ausführungsbeispiel kleiner als die zusammengenommene Öffnungsfläche der zweiten Öffnungen 26, 27. Alternativ kann die Öffnungsfläche der ersten Öffnung 25 größer sein als die Öffnungsfläche der zusammengenommenen Öffnungsflächen der zweiten Öffnungen 26, 27 oder die Öffnungsfläche der ersten Öffnung 25 kann gleich den zusammengenommenen Öffnungsflächen der zweiten Öffnungen 26, 27 sein.

Durch den Kanal 22 und durch das Lumen 23 erstreckt sich eine elektrische Leitung 60, die mit einer vorzugsweise in dem Primärstromkanalabschnitt 33 befestigten Elektrode 61 verbunden ist. Die Leitung 60 ist an dem HF-Generator 19 zur Erzeugung einer hochfrequenten Wechselspannung angeschlossen, um die Elektrode 61 kontinuierlich oder gepulst mit HF-Leistung zu speisen und ein Plasma, bspw. ein Argon-plasma, zwischen der Elektrode 61 des Instruments 13 und dem Gewebe 62 zu erzeugen. In den in den Figuren dargestellten Ausführungsformen weist das Instrument 13 jeweils eine einzige Elektrode 61 auf. Alternativ können auch mehr als eine Elektrode 61 in der ersten Öffnung 25 oder innerhalb des Instrumentenkopfes 17 vor der ersten Öffnung 25 angeordnet sein (nicht dargestellt). Beispielsweise können wenigstens zwei Elektroden in einem Kanalabschnitt 33 angrenzend an die erste Öffnung 25 angeordnet sein, und/oder es können wenigstens zwei erste Öffnungen 25 in dem Instrumentenkopf 17 angeordnet sein, um einen gemeinsamen Primärstrom 37 zu erzeugen, wobei in Kanalabschnitten vor den ersten Öffnungen 25 jeweils mindestens eine Elektrode 61 angeordnet ist.

Die Elektrode 61 ist vorzugsweise mittig und zumindest teilweise in dem Primärstromkanalabschnitt 33 angeordnet. Das distale Ende 63 der Elektrode 61 kann beispielsweise, wie dargestellt, mit der ersten Öffnung 25 abschließen oder alternativ aus der ersten Öffnung 25 herausschauen oder innerhalb des Primärstromkanalabschnitts 33 angeordnet sein.

Das proximale Ende 64 der Elektrode 61 kann beispielsweise, wie dargestellt, innerhalb des Primärstromkanalabschnitts 33, hinter dem Eingang 34 in den Primärstromkanalabschnitt 33 angeordnet sein, so dass das proximale Ende 64 der Elektrode 61 auf den Eingang 34 in den Primärstromkanalabschnitt 33 in Strömungsrichtung 40, gesehen folgt. Alternativ kann das proximale Ende 64 zwischen den zweiten Öffnungen 26, 27 oder vor den zweiten Öffnungen 26, 27 angeordnet sein, so dass die zweiten Öffnungen 26, 27 in Strömungsrichtung 40 gesehen nach dem proximale Ende 64 der Elektrode angeordnet sind. Das proximale Ende 64 der Elektrode 61 kann bis in das Lumen 23 hineinreichen.

Die Elektrode 61 ist in dem Primärstromkanalabschnitt 33 vorzugsweise, wie dargestellt, so orientiert angeordnet, dass sich die Elektrode 61 mit ihrer von einer seitlichen Kante 65 der Elektrode 61 bis zu der gegenüberliegenden seitlichen Kante 66 gemessenen Breite entlang der Öffnungsbreite 51 der ersten Öffnung 25 erstreckt. Die Elektrode 61 ist vorzugsweise, wie dargestellt, derart in dem Instrumentenkopf 17 orientiert angeordnet, dass sich die Elektrode 61 mit ihrer Breite entlang der durch die Öffnungsbreite 55 der zweiten Öffnung 26, 27 bestimmten seitlichen Ausdehnung der Sekundärströme 38, 39 erstreckt.

Bei der Elektrode 61 handelt es sich vorzugsweise um eine plättchen-, spatel-, nadel- oder messerförmige Elektrode 61. Die Elektrode 61 kann ein spitz zulaufendes distales Ende aufweisen.

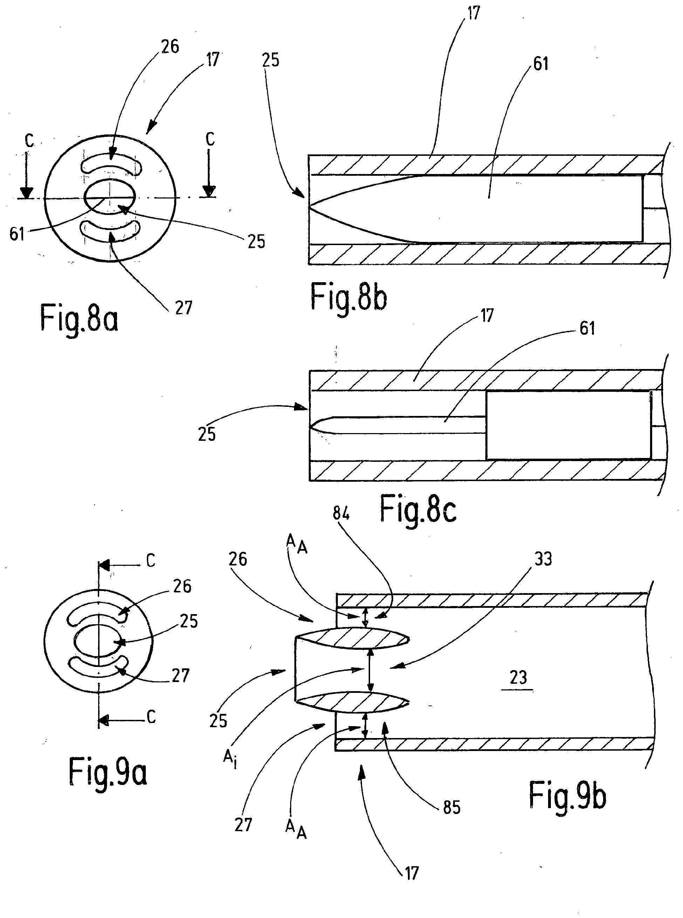

Die Elektrode 61 ist in dem Instrumentenkopf vorzugsweise befestigt, indem sich ein plättchen-, spatel- oder messerförmiger Abschnitt der Elektrode 61 auf Grund eines Übermaßes des Abschnitts der Elektrode 61 verglichen mit einem Innenmaß eines Abschnitt des Kanals 22, beispielsweise des Primärstromkanalabschnitts 33 und/oder des Lumens 23, auf gegenüberliegenden Seiten gegen die Innenwandfläche des Kanals 22, beispielsweise des Primärstromkanalabschnitts 33 und/oder des Lumens 23, stemmt. Dies ist in Fig. 8b und Fig. 8c beispielhaft dargestellt, die Teilschnittdarstellungen des Instrumentenkopfes 17 entlang der Schnittlinie C-C in Fig. 8a zeigen. Fig. 8b zeigt eine plättchenförmige Elektrode 61 mit spitz zulaufendem distalen Ende. In Fig. 8c ist eine Elektrode mit einem nadelförmigen distalen Abschnitt und einem plättchenförmigen proximalen Abschnitt zur Befestigung der Elektrode 61 dargestellt.

Der Strömungsweg von dem Lumen 23 zu den seitlichen zweiten Öffnungen 26, 27 ist vorzugsweise frei von Elektroden, um turbulente Gasströmungen zu verhindern.

Der Kanal 22 ist dazu eingerichtet, dass von der Gasversorgungseinheit 18 bereitgestellte Gas, beispielsweise Inertgas, insbesondere Argon, durch den Kanal 22 in das Lumen 23 des Kanals 22 zu den Öffnungen 25, 26, 27 zu leiten, wo das Gas aus der ersten Öffnung 25 als Primärstrom 37 und aus den zweiten Öffnungen 26, 27 als periphere Sekundärströme 38, 39 austritt, die neben und/oder wenigstens teilumfänglich um den Primärstrom 37 herum von dem Instrumentenkopf 17 wegströmen, um den Primärstrom 37 durch die Sekundärströme 38, 39 zu begleiten und/oder zumindest teilumfänglich einzuhüllen.

Der Strömungsquerschnitt des Primärstromkanalabschnitts 33 ist sich in Richtung zu der ersten Öffnung 25 verjüngend ausgebildet. Da der Strömungsquerschnittsflächeninhalt des Primärstromkanalabschnitts 33 entsprechend in Strömungsrichtung 40 abnimmt, kommt es in dem Primärstromkanalabschnitt 33 zu einer Beschleunigung des durch den Primärstromkanalabschnitt 33 in Richtung zu der ersten Öffnung 25 strömenden Gases vor dem Austritt aus der ersten Öffnung 25.

Die erfindungsgemäße Vorrichtung 10 arbeitet wie folgt:
Zum Zünden des Plasmas 69 (Fig. 1) bringt der Benutzer den Instrumentenkopf 17 in die Nähe des zu behandelnden Gewebes 62 und aktiviert die Gasversorgungseinheit 18, so dass Gas durch den Kanal 22 in Strömungsrichtung 40 von dem Eingang 70 in das Lumen 23 in dem Kopf 17 in das Lumen 23 des Instrumentenkopfes 17 strömt. Die Gasversorgungseinheit 18 kann beispielsweise dazu eingerichtet sein, das Lumen 23 mit einem Gasfluss zwischen mindestens 0,3 Litern Gas pro Minute und maximal 5 Litern Gas pro Minute, beispielsweise 1,5 Litern Gas pro Minute, zu speisen. Der zugeführte Gasstrom strömt in Strömungsrichtung 40 durch das Lumen 23 und wird anschließend in dem Instrumentenkopf 17 in einen zentralen Primärstrom 37, der an der Zündelektrode 61 vorbeigeführt wird, und einen oder mehrere Sekundärströme 38, 39 von Gas aufgeteilt, die auf gegenüberliegenden Seiten neben dem Primärstrom 37, den Primärstrom 37 zumindest teilumfänglich umgebend von dem Instrumentenkopf 17 wegströmen. Die Sekundärströme 38, 39 und der Primärstrom 37 bestehen aus dem gleichen Gas, da die erste Öffnung 25 und die zweiten Öffnungen 26, 27 von demselben innerhalb des Instrumentenkopfes 17 angeordneten Lumen 23 gespeist werden.

Durch die Beschleunigung des Primärstroms 37 aufgrund des sich verjüngenden Primärstromkanalabschnitts 33 sinkt der statische Druck im Primärstrom 37. Mit dem Primärstromkanalabschnitt 33 mit konvergierenden Wänden, der eine Düse bildet, kann das Gas für den Primärstrom 37 gegenüber dem Gas der Sekundärströme 38, 39 beschleunigt werden, so dass an der ersten Öffnung 25 der Primärstrom 37 eine höhere Strömungsgeschwindigkeit aufweist als die Sekundärströme 38, 39 auf beiden gegenüberliegenden Seiten jeweils aufweisen. Mit dem erfindungsgemäßen Instrument 13 kann demnach ein zweistufiges Geschwindigkeitsprofil des Gasstroms 37, 38, 39 weg von dem Instrumentenkopf 17 unter Verwendung nur eines Arbeitsmediums (Gases) erzielt werden. Das Geschwindigkeitsprofil kann beispielsweise auf Höhe der ersten Öffnung 25 an der ersten Öffnung 25 gemessen werden. Durch das Druckgefälle können die Sekundärströme 38, 39 zu dem Primärstrom 37 hingezogen werden.

Die Sekundärströme 38, 39 strömen außen an der Kanalwand 75 des Primärstromkanalabschnitts 33 und außen an der ersten Öffnung 25 vorbei. An dem Rand der ersten Öffnung 25 angesammelte Feuchtigkeit oder Flüssigkeit werden von dem Primärstrom 37 und dem den Bereich des Instrumentenkopfes 17 um die erste Öffnung 25 vorzugsweise streifenden Sekundärstrom 38, 39 weggeblasen oder aufgenommen, wobei ein Aerosol entstehen kann, dass von dem Primärstrom 37 bzw. dem Sekundärstrom 38, 39 weggetragen wird. Die entfernte Feuchtigkeit oder Flüssigkeit kann somit den Zündabstand nicht mehr beeinträchtigen.

Wenn die erste Öffnung 25 ausreichend nah an dem Gewebe 62 angeordnet ist, kann sich durch Aktivieren der HF-Versorgung der Elektrode 61 durch den HF-Generator 19 zwischen der Elektrode 61 und dem Gewebe 62 in dem Primärstrom 37 ein Zündfunke ausbilden, der das Plasma zündet. Das Plasma bildet sich dabei vor allem im Bereich des Primärstroms 37 durch Ionisation des Gases des Primärstroms 37 aus. Bereiche der Sekundärströme 38, 39 an dem Primärstrom 37 können ebenfalls ionisiert werden. Weiter außen strömendes Gas kann trotz des ausgebildeten zentralen Plasmastroms 37 nicht-ionisiert bleiben, so dass mit den zweiten Öffnungen 26, 27 ein nicht-ionisierter Strom von Gas neben bzw. wenigstens teilumfänglich um den Plasmastrom 37 erzeugt werden kann.

Damit die Plasmazündung gelingen kann, muss die erste Öffnung 25 an der Stirnseite 21 des Instrumentenkopfes 17 wenigstens bis zu einem Zündabstand 78 an das Gewebe 62 herangeführt werden. Der Zündabstand 78 beträgt in der Regel einige Millimeter. Ein großer Zündabstand 78 ist wünschenswert, da bei einem kleinen Zündabstand 78 einerseits die Gefahr steigt, dass der Instrumentenkopf 17 z.B. durch Berühren des Gewebes 62 verschmutzt wird, und andererseits der mit dem Plasma 69 zugleich behandelbare Gewebebereich relativ gering ist.

Es wird angenommen, dass bei dem erfindungsgemäßen Instrument 13 die Aufweitung des Primärstroms 37 in Querrichtung und die Vermischung mit dem nicht-inerten, feuchten Umgebungsmedium nach dem Austritt aus der ersten Öffnung 25 am Instrumentenkopf 17 durch den den Primärstrom 37 begleitenden, neben dem Primärstrom 37 strömenden und dabei den Primärstrom 37 vorzugsweise zumindest teilumfänglich einhüllenden Sekundärstrom 38, 39 vermindert wird, wodurch die Arbeitsgaskonzentration des Primärstroms 37 im Vergleich zu einem Instrument 13, das keinen Sekundärstrom 38, 39 bereitstellt, weniger schnell abnimmt. Es wird außerdem angenommen, dass Gasteilchen von dem Sekundärstrom 38, 39 aus der zweiten Öffnung 26, 27 auf den Primärstrom 37 übergehen können. Es wird zudem angenommen, dass die Ausbildung eines Primärstroms 37 mit einer bei der ersten Öffnung 25 gemessenen Strömungsgeschwindigkeit, die größer ist als die ebenfalls auf Höhe der ersten Öffnung 25 gemessenen Strömungsgeschwindigkeit des Sekundärstroms zur Stabilisierung des Primärstroms 37 und dadurch zur Vergrößerung des Zündabstands 77 führt. Jedenfalls weist das erfindungsgemäße Instrument 13 einen großen Zündabstand 78 selbst in feuchter Umgebung auf, wie sie etwa in einem Hohlraum 14 des Körpers eines Patienten vorliegen kann.

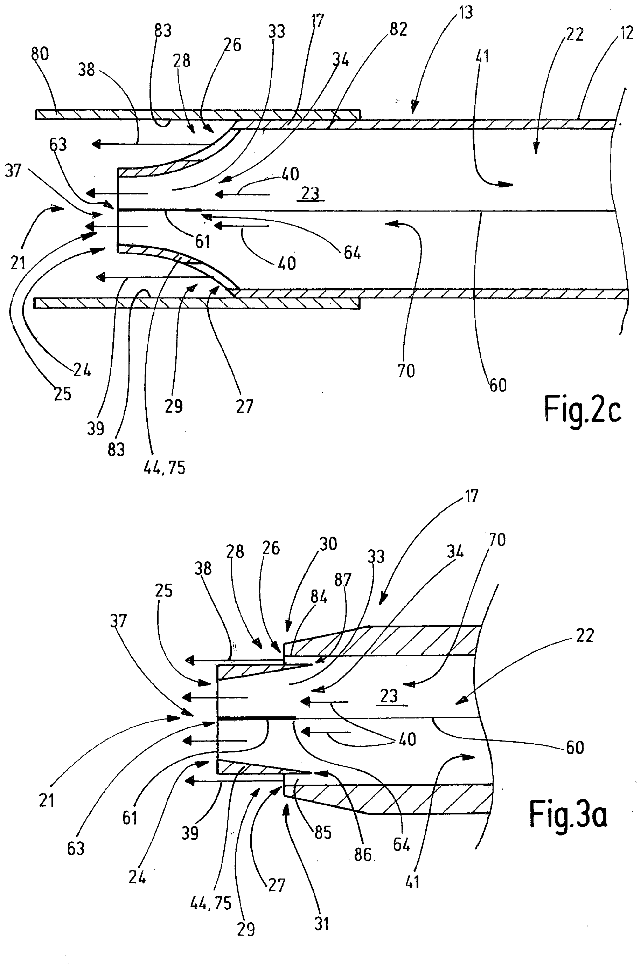

Fig. 2c zeigt die Ausführungsform gemäß Fig. 2a, 2b mit einem über die zweiten Öffnungen 26, 27 und vorzugsweise über die erste Öffnung 25 vorstehenden Führungsteil 80, das als Rohrelement ausgebildet ist, das auf das distale Ende 81 des Instrumentenkopfes 17 gesteckt ist.

Alternativ oder zusätzlich zu dem rohrförmigen Führungsteil 80 kann an den zweiten Öffnungen 26, 27 jeweils ein über die zweiten Öffnungen 26, 27 in Strömungsrichtung der Sekundärströme 38, 39 überstehendes, beispielsweise zungenartiges Führungsteil 80 an dem Instrumentenkopf angeordnet sein (nicht dargestellt). Die von dem Sondenkopf 17 weg weisenden Enden der Führungsteile können vor der ersten Öffnung enden, so dass die Führungsteile nicht über die erste Öffnung hinausstehen. Bei den zungenartigen Führungsteilen 80 kann es sich beispielsweise um jeweils einen Vorsprung der Wand 82 über die zweiten Öffnungen 26, 27 hinaus handeln, die das Lumen 23 nach außen begrenzt.

Das Führungsteil 80 ist dazu eingerichtet und angeordnet, dass die aus der zweiten Öffnung 26, 27 ausgetretenen Sekundärströme 38, 39 jeweils zwischen dem Führungsteil 80 und dem Primärstrom 37 bzw. dem Primärstromkanalabschnitt 33 strömen. Das vorstehende Führungsteil 80 grenzt den Sekundärstrom 38, 39 von dem Umgebungsmedium ab und dient dazu, das Expandieren von Gas des Sekundärstroms 38, 39 nach außen, weg von dem Primärstrom 37 hinauszuzögern, um zu einer Erhöhung des Zündabstands 78 beizutragen. Das Führungsteil 80 weist dazu eine radial nach innen weisende Führungswandfläche 83 auf, entlang der der Sekundärstrom 38, 39 strömt.

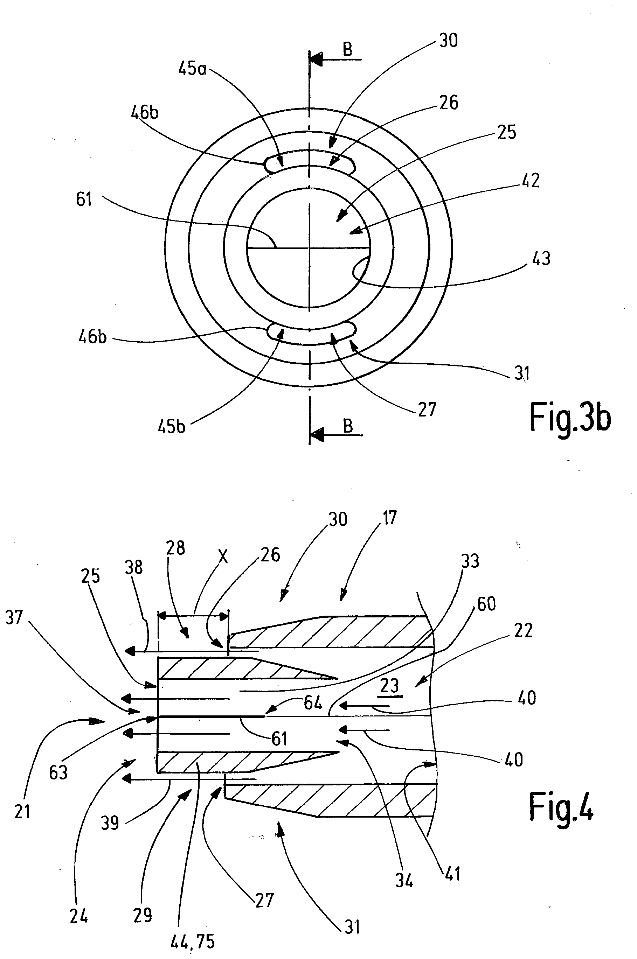

Fig. 3a zeigt einen Ausschnitt einer abgewandelten Ausführungsform des Kopfes 17 des erfindungsgemäßen Instruments 13 in einer Längsschnittansicht (Längsschnittdarstellung entlang der Schnittlinie B-B in Fig. 3b). Fig. 3b zeigt das Instrument der Fig. 3a in einer Ansicht von vorne. Wie aus Fig. 3a erkennbar, ist der Eingang 34 des Primärstromkanalabschnitts 33, der zwischen dem Lumen 23 und der ersten Öffnung 25 angeordnet ist, und das Lumen 23 mit der ersten Öffnung 25 verbindet, gegenüber den zweiten Öffnungen 26, 27 in Richtung entgegengesetzt der Strömungsrichtung 40 des Gases durch das Lumen 23 zurückversetzt angeordnet. Zwischen dem Primärstromkanalabschnitt 33 und der Wand 82 des Instrumentenkopfes 17, deren Wandabschnitt das Lumen 23 enthält, ist entsprechend vor jeder zweiten Öffnung 26, 27 jeweils ein in den peripheren Bereichen 28, 29 des Instrumentenkopfes 17 angeordneter Sekundärstromkanalabschnitt 83, 84 neben dem Primärstromkanalabschnitt 33 gebildet. Die voneinander gesonderten Sekundärstromkanalabschnitte 84, 85 grenzen jeweils an die entsprechende zweite Öffnung 26, 27 an. Die Sekundärstromkanalabschnitte 84, 85 können beispielsweise jeweils einen nieren-, bogen- oder sichelförmigen Querschnitt aufweisen. Die Sekundärstromkanalabschnitte 84, 85 sind vorzugsweise frei von Elektroden. Insbesondere sind in den zweiten Öffnungen 26, 27 vorzugsweise keine Elektroden angeordnet. Der Primärstromkanalabschnitt 33 separiert das durch den Primärstromkanalabschnitt 33 strömende Gas vorzugsweise von dem Eingang 34 des Primärstromkanalabschnitts 33 bis zu der ersten Öffnung 25 seitlich von dem Gas, das durch die Sekundärgaskanalabschnitte 84, 85 strömt, und dem Medium außerhalb des Sondenkopfes 17, bis das Gas aus der ersten Öffnung 25 heraustritt. Die Kanalwand des Primärstromkanalabschnitts 33, die den Primärstromkanalabschnitt 33 begrenzt, ist zwischen dem Eingang 34 des Primärstromkanals 33 bis zu der ersten Öffnung 25 entsprechend vorzugsweise frei von Öffnungen. Alternativ oder zusätzlich gilt für beide Sekundärstromkanalabschnitte 84, 85 jeweils vorzugsweise, dass die Kanalwand des Sekundärstromkanalabschnitts 84, 85, die den Sekundärstromkanalabschnitt 84, 85 begrenzt, vorzugsweise zwischen dem Eingang in den Sekundärstromkanalabschnitt 84, 85 bis zu der zweiten Öffnung 26, 27 frei von Öffnungen ist, um das durch die Sekundärstromkanalabschnitte 84, 85 strömende Gas jeweils seitlich zu separieren.

Der Instrumentenkopf 17 ist demnach ausgebildet, das durch das Lumen 23 strömende Gas innerhalb des Instrumentenkopfes 17 in wenigstens einen zentralen Primärstrom 37 durch den Primärstromkanalabschnitt 33 und zwei periphere Sekundärströme 38, 39 durch auf gegenüberliegenden Längsseiten des Primärstromkanalabschnitts 33 in dem Instrumentenkopf 17 angeordneten Sekundärstromkanalabschnitte 84, 85 aufzuteilen. Die Sekundärstromkanalabschnitte 84, 85 sind neben dem Primärstromkanalabschnitt 33 und, wie dargestellt, den Primärstromkanalabschnitt 33 zumindest teilumfänglich umgeben ausgebildet.

Der Instrumentenkopf 17 ist entsprechend derart ausgebildet, dass der Ort des Aufteilens des Gasstroms in den Primärstrom 37 und die Sekundärströme 38, 39 gegenüber der zweiten Öffnung 26, 27 entgegen der Strömungsrichtung 40 zurückversetzt ist.

Das Aufteilen des Stroms durch das Lumen 23 erfolgt an einer Aufteilungskante 86 innerhalb des Instrumentenkopfes 17 am Eingang 34 des Primärstromkanalabschnitts 33, die vorzugsweise schneidenartig entgegen die Strömungsrichtung 40 zulaufend ausgebildet ist.

Die Sekundärstromkanalabschnitte 84, 85 dienen der Führung des Sekundärstroms 38, 39 vor den Austritt aus der zweiten Öffnung 26, 27. In dem in Fig. 3a dargestellten Ausführungsbeispiel bleiben die Strömungsquerschnitte der Sekundärstromkanalabschnitte 84, 85 von dem Ort der Aufteilung bis zu der jeweiligen zweiten Öffnung 26, 27 konstant. Beispielsweise kann jeder Sekundärstromkanalabschnitt 84, 85 eine Länge von größer 0 Millimeter bis maximal 30 Millimeter, insbesondere größer 0 Millimeter bis maximal 10 Millimeter, vorzugsweise größer 0 Millimeter bis maximal 5 Millimeter aufweisen.

Die Aufteilungskante 86 kann an einem innerhalb des Kopfes 17 entgegen die Strömungsrichtung 40 in den Kanal 22 ragenden, beispielsweise zungenartigen, Aufteilungsvorsprung angeordnet sein (nicht dargestellt), der zusätzlich oder alternativ zu dem Primärstromkanalabschnitt 33 und/oder dem Sekundärstromkanalabschnitt 84, 85 in dem Instrumentenkopf 17 angeordnet ist. Der Abteilungsvorsprung ist innerhalb des Instrumentenkopfes 17 zwischen dem Primärstrom 37 und dem Sekundärstrom 38, 39 angeordnet. Die beschriebene Aufteilungskante 86 kann an dem Aufteilungsvorsprung angeordnet sein.

In dem Ausführungsbeispiel gemäß Fig. 3a, 3b ist der Primärstromkanalabschnitt 33 wie auch in dem Ausführungsbeispiel gemäß Fig. 3a, 3b derart ausgebildet, dass die Strömungsquerschnittsfläche des Primärstromkanalabschnitts 33 in Strömungsrichtung 40 aus dem Lumen 23 zu der ersten Öffnung 25 abnimmt, so dass das Gas in dem Primärstromkanalabschnitt 33 beschleunigt wird.

Wie aus Fig. 3b ersichtlich umschließen die zweiten Öffnungen 26, 27 und die Sekundärstromkanalabschnitte 84, 85 den Primärstromkanalabschnitt 33 jeweils teilumfänglich, bogenförmig. Die erste Öffnung 25 ist in dem Ausführungsbeispiel gemäß Fig. 3a und 3b kreisförmig. Die die zweiten Öffnungen 26, 27 radial begrenzenden Konturen sind kreisbogenförmig. Die erste Öffnung 25 kann abweichend davon beispielsweise eine abgeflacht mehreckige oder abgeflacht gerundete, z.B. ovale, Form aufweisen. Die zweiten Öffnungen 26, 27 und die Sekundärstromkanalabschnitte 84, 85 (im Querschnitt) können jeweils eine von der Kreisbogenform abweichende Form aufweisen, um den Primärstromkanalabschnitt 33 teilumfänglich zu umschließen. Die Öffnungsflächen 45a, 45b der zweiten Öffnungen 26, 27 sind eben und werden jeweils durch eine Endkante 46 des Sekundärstromkanalabschnitts 84, 85 begrenzt. In dem Ausführungsbeispiel der Fig. 3a weisen die Öffnungsflächen 45a, 45b der zweiten Öffnungen 26, 27 im Wesentlichen in dieselbe Richtung von dem Instrumentenkopf 17 weg wie die Öffnungsfläche 42 der ersten Öffnung 25. Die Beschreibung zu der Ausführungsform gemäß Fig. 1, 2a und 2b gilt ansonsten analog auch für die Ausführungsform gemäß Fig. 3a, 3b.

Bevorzugt ist der Instrumentenkopf 17 derart ausgebildet, dass dieser einen oder mehrere Sekundärströme 38, 39 neben dem Primärstrom 37 oder um den Primärstrom 37 herum erzeugt, wobei der oder die Sekundärströme 38, 39 gemessen an der ersten Öffnung 25 eine höhere Strömungsgeschwindigkeit als der Primärstrom 37 gemessen an der ersten Öffnung 25 aufweisen. Der Instrumentenkopf 17 kann dazu beispielsweise zur Beschleunigung des Gases für den oder die Sekundärströme 38, 39 und/oder zur Verminderung der Geschwindigkeit des Gases für den Primärstrom 37 eingerichtet sein. Zur Verminderung der Geschwindigkeit des Gases für den Primärstrom können beispielsweise Elemente, wie etwa ein Gitter und/oder eine oder mehrere Lamellen, im Inneren des Instrumentenkopfes 17 im Strömungsweg des Gases für den Primärstrom angeordnet sein. Mit Hilfe des Instrumentenkopfes 17 kann ein zweistufiges Geschwindigkeitsprofil des Gasstroms 37, 38, 39 weg vom Instrumentenkopf 17 unter Verwendung nur eines Arbeitsmediums (Gases) erzielt werden. Dadurch kann zwischen dem Primärstrom 37 und dem oder den Sekundärströmen 38, 39 ein Druckgefälle bestehen, wodurch der Primärstrom 37 zu dem Sekundärstrom 38 oder den Sekundärströmen 39 hingezogen werden kann.

Fig. 4 zeigt einen Ausschnitt einer beispielhaften Ausführungsform des erfindungsgemäßen Instruments 13 mit dem Instrumentenkopf 17, bei dem die Strömungsquerschnittsflächen der Sekundärstromkanalabschnitte 84, 85, in Abwandlung zu der Ausführungsform gemäß Fig. 3a, in Strömungsrichtung 40 von dem Eingang 87 des Sekundärstromkanalabschnitts 84, 85 in Richtung zu der zweiten Öffnung 26, 27 abnehmen. Dadurch wird das Gas, das von dem Lumen 23 durch den Sekundärstromkanalabschnitt 84, 85 zu der zweiten Öffnung 26, 27 strömt, in dem Sekundärstromkanalabschnitt 84, 85 beschleunigt. In der dargestellten Ausführungsform bleibt der Strömungsquerschnitt des Primärstromkanalabschnitts 33 vom Eingang 34 des Primärstromkanalabschnitts 33 bis zur ersten Öffnung 25 konstant. Eine Abwandlung ist möglich, in der sowohl die Strömungsquerschnittsfläche des Primärstromkanalabschnitts 33 als auch die Strömungsquerschnittsflächen der Sekundärstromkanalabschnitte 84, 85 in Richtung zu den Öffnungen 25, 26, 27 abnehmen, um das Gas vor dem Durchtritt durch die erste Öffnung 25 bzw. durch die zweiten Öffnungen 26, 27 zu beschleunigten. Der Instrumentenkopf 17 kann durch die Beschleunigung des Gases in den Sekundärstromkanalabschnitten 84, 85 beispielsweise derart ausgebildet sein, dass die Strömungsgeschwindigkeiten der Sekundärströme 38, 39 an der ersten Öffnung größer sind als die Strömungsgeschwindigkeit des Primärstroms 37 an der ersten Öffnung.

Ansonsten gilt für die Ausführungsform gemäß Fig. 4 die Beschreibung zu der Ausführungsform gemäß Fig. 3a analog. Eine beispielhafte Ansicht der Ausführungsform gemäß Fig. 4 von vorne bietet Fig. 3b.

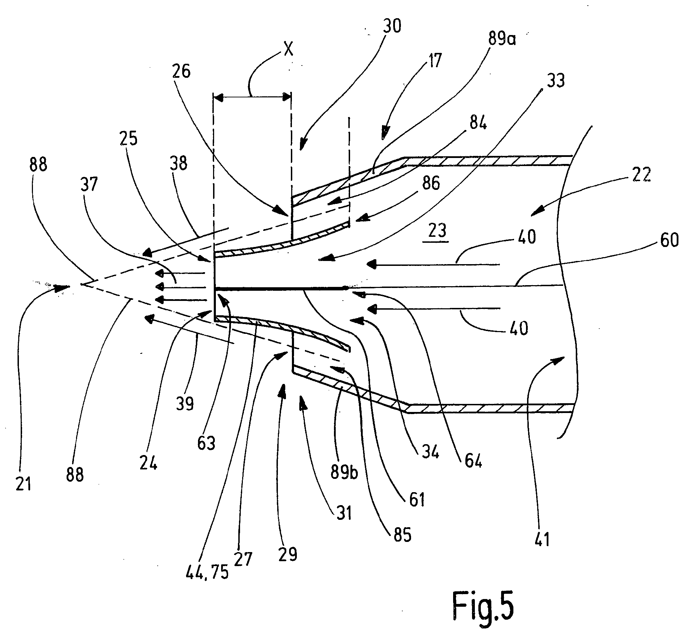

Fig. 5 zeigt einen Ausschnitt des erfindungsgemäßen Instruments 13 mit einem Instrumentenkopf 17 gemäß einer abgewandelten Ausführungsform in einer Längsschnittansicht. In dieser Ausführungsform sind die Sekundärstromkanalabschnitte 84, 85 so ausgebildet, dass diese die Sekundärströme auf beiden Seiten des Primärstroms auf den Primärstrom in dem Primärstromkanalabschnitt 33 und/oder außerhalb des Primärstromkanalabschnitts 33 hinter der ersten Öffnung 25 richten, wie durch die gestrichelten Linien 88 angedeutet, die die Strömungsrichtungen der Sekundärströme 38 aus den zweiten Öffnungen 26, 27 angeben. Die Strömungsrichtungen ergeben sich aus der Anordnung der die Sekundärstromkanalabschnitte 84, 85 begrenzenden Wandflächen der Sekundärstromkanalabschnitte 84, 85. Die Sekundärstromkanalabschnitte 84, 85 können derart ausgerichtet angeordnet sein, dass die Sekundärströme 38, 39 auf den Öffnungsrand der ersten Öffnung 25 ausgerichtet werden. Damit kann erreicht werden, dass eine große Menge des Gases der Sekundärströme 38, 39 den Öffnungsrand der ersten Öffnung streift.

In der Ausführungsform gemäß Fig. 5 konvergieren das Lumen 23 begrenzenden Wandabschnitte 89a, 89b des Instrumentenkopfes 17, so dass die Strömungsquerschnittsfläche des Lumens 23 in Richtung zu den Öffnungen 25, 26, 27 abnimmt. Auf Grund des in Strömungsrichtung 40 zu den Öffnungen 25, 26, 27 abnehmenden Strömungsquerschnitts des Lumens 23 wird durch das Lumen 23 strömendes Gas schon vor der Aufteilung in Primärstrom 37 und Sekundärstrom 38, 39 beschleunigt.

Ansonsten gilt die Beschreibung für die Ausführungsform gemäß Fig. 3a, 3b analog.

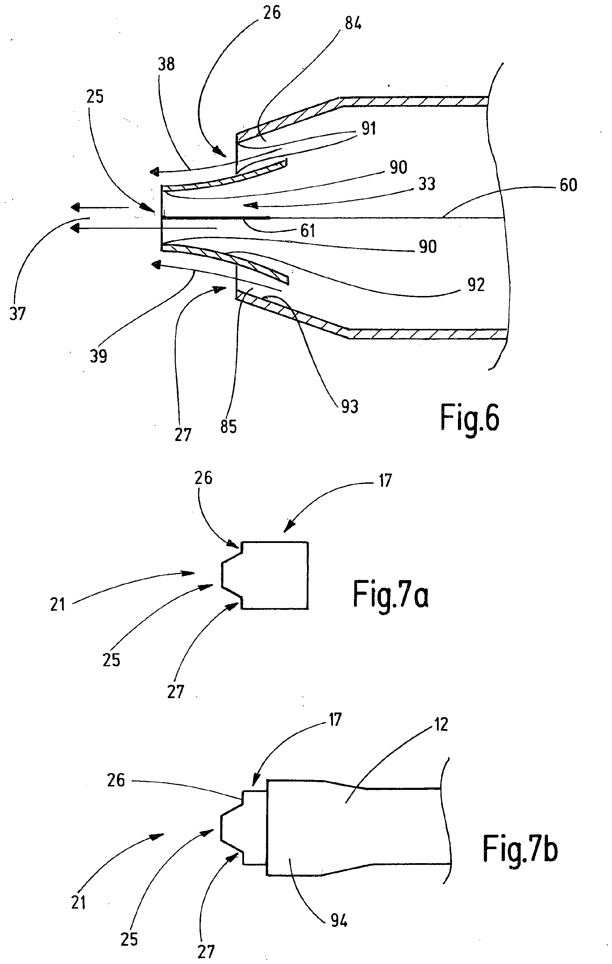

Fig. 6 zeigt einen Ausschnitt aus einem der Ausführungsform gemäß Fig. 5 entsprechenden Instrument 13 mit hydrophoben Wandflächen. Wandflächen der Ausführungsformen gemäß Fig. 1 bis 4 können entsprechende hydrophobe Wandflächen aufweisen:
Von Vorteil für das Wegblasen bzw. Wegtragen von wasserhaltiger Flüssigkeit von der ersten Öffnung 25 und/oder von der zweiten Öffnung 26, 27 durch die Sekundärströme 38, 39 und/oder den Primärstrom 37 ist, wenn die Oberfläche des Instrumentenkopfes 17 an der ersten Öffnung 25 und/oder an den zweiten Öffnungen 26, 27 hydrophob ist. Wenn die die erste Öffnung 25 nach Innen begrenzende Wandfläche 90 und/oder die die zweiten Öffnungen 26, 27 nach Innen begrenzenden Wandflächen 91 hydrophob sind, ist die Gefahr vermindert, dass sich Feuchtigkeit oder Flüssigkeit in der ersten Öffnung 25 bzw. in den zweiten Öffnungen 26, 27 sammelt und diese verstopft.

Vorzugsweise sind die den Primärstromkanalabschnitt 33 und/oder die den Sekundärstromkanalabschnitte 84, 85 nach innen begrenzenden Wandflächen 92, 93 hydrophob, um die Gefahr zu vermindern, dass sich Feuchtigkeit in dem Primärstromkanalabschnitt 33 bzw. in den Sekundärstromkanalabschnitten 84, 85 sammelt und diese verstopft.

Die Wandflächen 90-93 können, z.B. durch eine hydrophobe Beschichtung, hydrophob ausgebildet sein. Hydrophob ausgebildete Wandflächen 90-93 können beispielsweise aus PTFE sein oder eine PTFE-Beschichtung aufweisen. Der Sondenkopf 17 ist vorzugsweise aus Keramik mit hydrophober Oberfläche.

Fig. 7a zeigt einen erfindungsgemäßen Instrumentenkopf 17 in einer Profilansicht, beispielsweise aus Keramik, der ein von der Gaszuführungsleitung 12, die den Instrumentenkopf 17 zur Gasversorgung mit einer Gasversorgungseinheit 18 verbindet, gesondertes Teil ist. Fig. 7b zeigt den Instrumentenkopf 17 gemäß Fig. 7a, der an eine Gaszuführungsleitung 12, beispielsweise einen Schlauch oder ein flexibles Rohr, des Instruments 13 angeschlossen ist, wobei der Instrumentenkopf 17 beispielsgemäß in einem distalen Ende 94 der Zuführungsleitung 12 gefasst ist.

Alternativ zu einem Instrumentenkopf 17 als von der Gaszuführungsleitung 12 gesondertes Teil kann der Instrumentenkopf 17 ein Abschnitt an dem distalen Ende 94 einer Gaszuführungsleitung 12 sein. Ein erfindungsgemäßer Instrumentenkopf 17 kann beispielsweise hergestellt werden, indem der Mantel einer Leitung 12, beispielsweise eines Schlauchs oder eines flexiblen Rohres, an seinem Ende auf gegenüberliegenden Seiten quer zur Längserstreckungsrichtung der Leitung 12, radial bis zum dem von dem Mantel begrenzten Lumen eingeschnitten wird und der Abschnitt 12 am Ende der Leitung 12 zwischen den Schnitten einerseits und dem Ende der Leitung 12 andererseits durch Zusammendrücken abgeflacht wird. Dabei öffnen sich die zweiten Öffnungen 26, 27 an den Einschnitten. Die Öffnung an dem Ende der Leitung bildet die erste Öffnung 25, in die eine Elektrode eingesetzt werden kann. Die Gestalt des Instrumentenkopfes 17, z.B. aus Fig. 7a, 7b, kann dieser beschriebenen Form gleichen, der Kopf 17 kann jedoch aus Keramik bestehen.

Fig. 9b zeigt einen Längsschnitt durch einen erfindungsgemäßen Instrumentenkopf 17 (entlang der Schnittlinie C-C in Fig. 9a, Elektrode 61 und Zuleitung 60 sind nicht dargestellt). Eingezeichnet sind die Strömungsquerschnittsflächen Aᵢ des Primärstromkanalabschnitts 33 an seiner engsten Stelle sowie die Strömungsquerschnittsflächen A_{A} der Sekundärstromkanalabschnitte 84, 85 an deren engsten Stellen. Das Verhältnis der Summe der Strömungsquerschnittsflächen Aᵢ der Primärstromkanalabschnitte 33 an deren engsten Stellen zu der Summe der Strömungsquerschnittsflächen A_{A} der Sekundärstromkanalabschnitte 84, 85 an deren engsten Stellen ist vorzugsweise maximal 3 zu 1 bis minimal 1 zu 3. Vorzugsweise ist die Summe der Strömungsquerschnittsflächen Aᵢ der ersten Primärstromkanalabschnitte 33 an deren engsten Stellen größer als die Summe der Strömungsquerschnittsflächen A_{A} der Sekundärstromkanalabschnitte 84, 85 an deren engsten Stellen. Die Sekundärstromkanalabschnitte 84, 85 und/oder die Primärstromkanalabschnitte 33 können derart ausgebildet sein, dass das in den Kanalabschnitten strömende Gas Schallgeschwindigkeit erreicht.

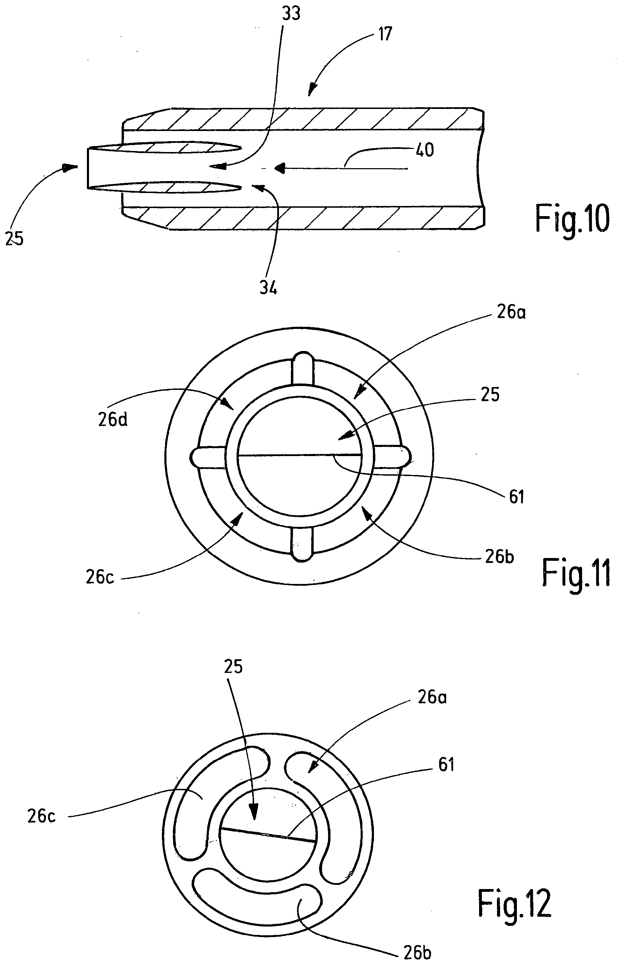

Fig. 10 zeigt eine Ausführungsform des Instrumentenkopfs 17 des erfindungsgemäßen Instruments 13 in einer Längsschnittdarstellung (die Elektrode 61 und deren Zuleitung 60 sind nicht dargestellt). Die Wand des Primärstromkanalabschnitts 33 ist, wie dargestellt, derart gestaltet, dass die bei dem Längsschnitt entstehenden beiden Schnittflächen (Längsschnitt entlang der Strömungsrichtung 40 mittig durch den Instrumentenkopf 17) der Wand des Primärstromkanalabschnitts 33 jeweils eine Tropfenform aufweisen. Der Primärstromkanalabschnitt 33 ist derart gestaltet, dass die quer zur Strömungsrichtung gemessene Abmessung jeder Längsschnittfläche vom gerundeten proximalen Ende (am Eingang 34) des Primärstromkanalabschnitts 33 in Richtung erster Öffnung 25 zunächst zunimmt und dann derart stromabwärts derart abnimmt, dass der Längsschnitt in Richtung zu dem distalen Ende des Primärstromkanalabschnitts 33 spitz zuläuft. Wenn der Primärstromkanalabschnitt 33 eines erfindungsgemäßen Instrumentenkopfs 17 derart gestaltet ist, das die quer zur Strömungsrichtung 40 gemessene Abmessung der Längsschnittfläche stromabwärts zu dem distalen Ende des Primärstromkanalabschnitts 33 derart abnimmt, dass der Längsschnitt spitz zuläuft, kann Feuchtigkeit besonders effektiv von der ersten Öffnung 25 weggeblasen werden.

Fig. 11 und Fig. 12 zeigen Ausführungsformen des Instrumentenkopfs 17 von vorne mit mehreren auf einem Umfang um den Primärstromkanalabschnitt 33 angeordneten zweiten Öffnungen 26a-26d bzw. 26a - 26c. Die Anordnung der zweiten Öffnungen des erfindungsgemäßen Instrumentenkopfs 17 kann symmetrisch bzgl. der Ebene sein, die sich entlang der Breite der Elektrode und deren Länge erstreckt. Fig. 12 zeigt eine Alternative dazu, in der die Anordnung der zweiten Öffnungen 26a - 26c nicht-symmetrisch bzgl. der Elektrodenebene ist.

Es wird ein neues Instrument 13 zur elektrochirurgischen Behandlung beschrieben. Das Instrument 13 weist einen Instrumentenkopf 17 auf. Der Instrumentenkopf 17 bildet einen Leitungskörper mit einem mit Gas beaufschlagbaren Lumen 23. Das Lumen 23 ist an wenigstens eine erste Öffnung 25 und wenigstens eine zweite Öffnung 26, 27 in dem Instrumentenkopf 17 angeschlossen, aus denen das Gas bei Beaufschlagung des Lumens 23 mit Gas aus dem Instrumentenkopf 17 austritt. Durch die erste Öffnung 25 tritt ein Primärstrom 37 von Gas aus dem Instrumentenkopf 17 in Richtung zu dem Gewebe 62 aus. Die zweite Öffnung 26, 27 ist derart bezüglich der ersten Öffnung 25 angeordnet, dass aus der zweiten Öffnung 26, 27 ein Sekundärstrom 38, 39 austritt, der neben dem Primärstrom 37 und/oder, ggf. mit weiteren Sekundärströmen 38, 39 aus weiteren zweiten Öffnungen 26, 27, den Primärstrom 37 zumindest teilumfänglich umgebend von dem Instrumentenkopf 17 wegströmt. Der Sekundärstrom 38, 39 hüllt den Primärstrom 37 vorzugsweise ein. In dem Strömungsweg des Gases von dem Inneren des Instrumentenkopfes 17 aus der ersten Öffnung 25 heraus ist eine Elektrode 61 angeordnet, um ein Plasma zwischen der ersten Öffnung 25 und dem zu behandelnden Gewebe 62 zu zünden. Durch den den Primärstrom 37 begleitend ausgebildeten Sekundärstrom 38, 39 gelingt das Zünden des Plasmas auch, wenn das Instrument 13 einen relativ großen Abstand 78 zu dem zu behandelnden Gewebe 62 aufweist. Wenn der Primärstrom 37 mit dem erfindungsgemäßen Instrument als Kernstrom aus der ersten Öffnung 25 und der Sekundärstrom 38, 39 als Mantelstrom aus den zweiten Öffnungen 26, 27 ausgebildet wird, gelingt die Zündung des Plasmas bereits bei relativ großen Abständen des Instruments 13 zum Gewebe 62. Kernstrom 37 und Mantelstrom 38, 39 werden aus demselben Lumen 23 gespeist, so dass diese aus dem gleichen Gas bestehen.

**Bezugszeichenliste:**

| | |
|---|---|
| 10 | Vorrichtung |
| 11 | Endoskop |
| 12 | Gaszuführungsleitung |
| 13 | Instrument |
| 14 | Hohlraum (Lumen) des Körpers |
| 15 | Längserstreckungsrichtung |
| 16 | Distales Ende des Endoskops |
| 17 | Kopf |
| 18 | Gasversorgungseinheit |
| 19 | HF-Generator |
| 21 | Stirnseite |
| 22 | Kanal |
| 23 | Lumen |
| 24 | Zentraler Bereich |
| 25 | Erste Öffnung |
| 26, 26a 26d | Zweite Öffnung |
| 27 | Zweite Öffnung |
| 28 | Peripherer Bereich |
| 29 | Peripherer Bereich |
| 30 | Seite des Instrumentenkopfes |
| 31 | Seite des Instrumentenkopfes |
| 32 | Mantelfläche |
| 33 | Primärstromkanalabschnitt |
| 34 | Eingang |
| 37 | Primärstrom/Plasmastrom |
| 38 | Sekundärstrom |
| 39 | Sekundärstrom |
| 40 | Strömungsrichtung |
| 41 | Proximales Ende |
| 42 | Öffnungs fläche |
| 43 | Endkante |
| 44 | Wand |
| 45a, 45b | Öffnungs flächen |
| 46a, b | Kante |
| 51 | Öffnungsbreite |
| 52 | Öffnungshöhe |
| 53 | Seite |
| 54 | Seite |
| 55 | Öffnungsbreite |
| 56 | Öffnungshöhe |
| 60 | Elektrische Leitung |
| 61 | Elektrode |
| 62 | Gewebe |
| 63 | Distales Ende der Elektrode |
| 64 | Proximales Ende der Elektrode |
| 65 | Kante |
| 66 | Kante |
| 69 | Plasma |
| 70 | Eingang |
| 75 | Kanalwand |
| 78 | Abstand/Zündabstand |
| 80 | Führungsteil |
| 81 | Distales Ende |
| 82 | Wand |
| 82a, 82b | Wandabschnitte |
| 83 | Führungswandfläche |
| 84 | Sekundärstromkanalabschnitt |
| 85 | Sekundärstromkanalabschnitt |
| 86 | Aufteilungskante |
| 87 | Eingang |
| 88 | Linien |
| 89a, b | Wandabschnitte |
| 90-93 | Wandflächen |
| 94 | Distales Ende der Gaszuführungsleitung |
| x | Abstand |
| Aᵢ | Strömungsquerschnittsfläche |
| A_{A} | Strömungsquerschnittsfläche |
| A-A | Schnittlinie |
| B-B | Schnittlinie |
| C-C | Schnittlinie |

## Patentansprüche

1. Elektrochirurgisches Instrument (13) mit
einem Kopf (17) mit einem mit Gas beaufschlagbaren Lumen (23) eines Kanals (22) und einer an das Lumen (23) angeschlossenen ersten Öffnung (25) zur Ausbildung eines Primärstroms (37) von Gas weg von dem Kopf (17) und wenigstens einer an das Lumen (23) angeschlossenen zweiten Öffnung (26, 27) zur Ausbildung wenigstens eines Sekundärstroms (38, 39) von Gas neben dem Primärstrom (37) oder wenigstens teilumfänglich um den Primärstrom (37), so dass die einen oder mehreren Sekundärströme (38, 39), die neben dem Primärstrom (37) oder wenigstens teilumfänglich um den Primärstrom (37) ausgebildet sind, den Primärstrom (37) zumindest teilumfänglich umgeben, wobei in dem Kopf (17) eine Elektrode (61) angeordnet ist,
wobei die Elektrode (61) in einem an die erste Öffnung (25) angrenzenden, in die erste Öffnung (25) mündenden Kanalabschnitt (33) des Kanals (22) angeordnet ist, wobei das distale Ende (63) der Elektrode (61) in Gasströmungsrichtung (40) gesehen nach der zweiten Öffnung (26, 27) angeordnet ist.

2. Elektrochirurgisches Instrument (13) nach Anspruch 1, wobei die wenigstens eine zweite Öffnung (26, 27) von der ersten Öffnung (25) zurückversetzt angeordnet ist.

3. Elektrochirurgisches Instrument (13) nach einem der vorherigen Ansprüche, wobei der Kanal (22) einen Primärstromkanalabschnitt (33) aufweist, der an die erste Öffnung (25) angeschlossen ist und wobei der Strömungsquerschnitt des Primärstromkanalabschnitts (33) vor der ersten Öffnung (25) in Richtung zu der ersten Öffnung (25) abnimmt.

4. Elektrochirurgisches Instrument (13) nach einem der vorherigen Ansprüche, wobei der Eingang (34) des Primärstromkanalabschnitts (33) bündig mit der oder den zweiten Öffnungen abschließt oder wobei der Eingang (34) des Primärstromkanalabschnitts (33) von der oder den zweiten Öffnungen (26, 27) nach proximal zurückversetzt angeordnet ist.

5. Elektrochirurgisches Instrument (13) nach einem der vorherigen Ansprüche, wobei die Öffnungsfläche (42) der ersten Öffnungen (25) größer ist als die Öffnungsfläche (45a, 45b) der zweiten Öffnungen (26, 27).

6. Elektrochirurgisches Instrument (13) nach einem der vorherigen Ansprüche, wobei die Öffnungsbreite (51) der ersten Öffnung (25) vorzugsweise größer als die orthogonal zu der Öffnungsbreite (51) gemessene Öffnungshöhe (52) der ersten Öffnung (25) ist.

7. Elektrochirurgisches Instrument (13) nach einem der vorherigen Ansprüche, wobei die zweite Öffnung (26, 27) nierenförmig oder bogenförmig oder sichelförmig ist.

8. Elektrochirurgische Instrument (13) nach einem der vorherigen Ansprüche, wobei neben dem Primärstromkanalabschnitt (33) oder um den Primärstromkanalabschnitt (33) herum ein Sekundärstromkanalabschnitt (84, 85) angeordnet ist, der an die zweite Öffnung (26, 27) angeschlossen ist.

9. Elektrochirurgische Instrument (13) nach einem der vorhergehenden Ansprüche, wobei der Strömungsquerschnitt des Sekundärstromkanalabschnitts (84, 85) vor der zweiten Öffnung (26, 27) in Richtung zu der zweiten Öffnung (26, 27) abnimmt.

10. Elektrochirurgische Instrument (13) nach einem der vorherigen Ansprüche, wobei der Eingang (34) des Primärstromkanalabschnitts (33) eine Aufteilungskante (86) aufweist.

11. Elektrochirurgisches Instrument (13) nach einem der vorherigen Ansprüche, wobei das elektrochirurgische Instrument (13) ein monopolares Instrument ist.

12. Elektrochirurgisches Instrument (13) nach einem der vorherigen Ansprüche, wobei die Elektrode (61) eine plättchen-, spatel-, nadel- oder messerförmige Elektrode (61) ist.

## Claims

1. Electrosurgical instrument (13) with
a head (17) with a lumen (23) of a channel (22) which can be loaded with gas, and a first opening (25) connected to the lumen (23) for forming a primary stream (37) of gas away from the head (17), and at least one second opening (26, 27) connected to the lumen (23) for forming at least one secondary stream (38, 39) of gas next to the primary stream (37) or at least partially around the primary stream (37), so that the one or more secondary streams (38, 39), which are formed next to the primary stream (37) or at least partially around the primary stream (37), at least partially surround the primary stream (37), wherein an electrode (61) is arranged in the head (17),
wherein the electrode (61) is arranged in a channel portion (33) of the channel (22) adjoining the first opening (25) and opening into the first opening (25), wherein the distal end (63) of the electrode (61) is arranged downstream of the second opening (26, 27) viewed in the gas flow direction (40).

2. Electrosurgical instrument (13) according to claim 1, wherein the at least one second opening (26, 27) is arranged set back from the first opening (25).

3. Electrosurgical instrument (13) according to one of the preceding claims, wherein the channel (22) has a primary stream channel portion (33) which is connected to the first opening (25), and wherein the flow cross-section of the primary stream channel portion (33) upstream of the first opening (25) reduces in the direction towards the first opening (25).

4. Electrosurgical instrument (13) according to any of the preceding claims, wherein the inlet (34) of the primary stream channel portion (33) terminates flush with the second opening or openings, and wherein the inlet (34) of the primary stream channel portion (33) is arranged proximally set back from the second opening or openings (26, 27).

5. Electrosurgical instrument (13) according to any of the preceding claims, wherein the opening area (42) of the first openings (25) is larger than the opening area (45a, 45b) of the second openings (26, 27).

6. Electrosurgical instrument (13) according to any of the preceding claims, wherein the opening width (51) of the first opening (25) is preferably larger than the opening height (52) of the first opening (25) measured orthogonally to the opening width (51).

7. Electrosurgical instrument (13) according to any of the preceding claims, wherein the second opening (26, 27) is kidney-shaped or arcuate or sickle-shaped.

8. Electrosurgical instrument (13) according to any of the preceding claims, wherein a secondary stream channel portion (84, 85), which is connected to the second opening (26, 27), is arranged next to the primary stream channel portion (33) or around the primary stream channel portion (33).

9. Electrosurgical instrument (13) according to any of the preceding claims, wherein the flow cross-section of the secondary stream channel portion (84, 85) upstream of the second opening (26, 27) reduces in the direction towards the second opening (26, 27).

10. Electrosurgical instrument (13) according to any of the preceding claims, wherein the inlet (34) of the primary stream channel portion (33) has a dividing edge (86).

11. Electrosurgical instrument (13) according to any of the preceding claims, wherein the electrosurgical instrument (13) is a monopolar instrument.

12. Electrosurgical instrument (13) according to any of the preceding claims, wherein the electrode (61) is a plate-like, spatula-like, needle-like or knife-like electrode (61).

## Revendications

1. Instrument électrochirurgical (13), comprenant
une tête (17) dotée d'une lumière (23), pouvant recevoir du gaz, d'un canal (22) et d'une première ouverture (25) reliée à la lumière (23) et destinée à former un flux primaire (37) de gaz, partant de la tête (17), et d'au moins une deuxième ouverture (26, 27) reliée à la lumière (23) et destinée à former au moins un flux secondaire (38, 39) de gaz, à côté du flux primaire (37) ou au moins en partie autour du flux primaire (37), de sorte que le ou les flux secondaires (38, 39), qui sont formés à côté du flux primaire (37) ou au moins en partie autour du flux primaire (37), entourent au moins en partie le flux primaire (37), sachant qu'une électrode (61) est disposée dans la tête (17),
l'électrode (61) étant disposée dans une portion de canal (33) du canal (22) qui est adjacente à la première ouverture (25) et qui débouche dans la première ouverture (25), l'extrémité distale (63) de l'électrode (61) étant disposée en aval de la deuxième ouverture (26, 27), vu dans le sens d'écoulement du gaz (40).

2. Instrument électrochirurgical (13) selon la revendication 1, dans lequel la deuxième ouverture (26, 27), au nombre d'au moins une, est disposée en étant décalée vers l'arrière par rapport à la première ouverture (25).

3. Instrument électrochirurgical (13) selon l'une des revendications précédentes, dans lequel le canal (22) présente une portion de canal de flux primaire (33) qui est raccordée à la première ouverture (25), et dans lequel la section d'écoulement de la portion de canal de flux primaire (33) diminue devant la première ouverture (25), en direction de la première ouverture (25).

4. Instrument électrochirurgical (13) selon l'une des revendications précédentes, dans lequel l'entrée (34) de la portion de canal de flux primaire (33) est alignée avec la ou les deuxièmes ouvertures, ou dans lequel l'entrée (34) de la portion de canal de flux primaire (33) est disposée en étant décalée vers l'arrière, dans la direction proximale, par rapport à la ou aux deuxièmes ouvertures (26, 27).

5. Instrument électrochirurgical (13) selon l'une des revendications précédentes, dans lequel la surface d'ouverture (42) des premières ouvertures (25) est plus grande que la surface d'ouverture (45a, 45b) des deuxièmes ouvertures (26, 27).

6. Instrument électrochirurgical (13) selon l'une des revendications précédentes, dans lequel la largeur d'ouverture (51) de la première ouverture (25) est de préférence plus grande que la hauteur d'ouverture (52) de la première ouverture (25), mesurée de façon orthogonale à la largeur d'ouverture (51).

7. Instrument électrochirurgical (13) selon l'une des revendications précédentes, dans lequel la deuxième ouverture (26, 27) présente une forme de rein ou une forme d'arc ou une forme de croissant.

8. Instrument électrochirurgical (13) selon l'une des revendications précédentes, dans lequel il est prévu, à côté de la portion de canal de flux primaire (33) ou autour de la portion de canal de flux primaire (33), une portion de canal de flux secondaire (84, 85) qui est raccordée à la deuxième ouverture (26, 27).

9. Instrument électrochirurgical (13) selon l'une des revendications précédentes, dans lequel la section d'écoulement de la portion de canal de flux secondaire (84, 85) diminue devant la deuxième ouverture (26, 27), en direction de la deuxième ouverture (26, 27).

10. Instrument électrochirurgical (13) selon l'une des revendications précédentes, dans lequel l'entrée (34) de la portion de canal de flux primaire (33) présente une arête de division (86).

11. Instrument électrochirurgical (13) selon l'une des revendications précédentes, dans lequel l'instrument électrochirurgical (13) est un instrument monopolaire.

12. Instrument électrochirurgical (13) selon l'une des revendications précédentes, dans lequel l'électrode (61) est une électrode (61) en forme de plaquette, de spatule, d'aiguille ou de lame.
